# EUROPEAN PATENT SPECIFICATION

(11) **EP 4 145 138 B1**
(45) Date of publication and mention of the grant of the patent: **05.03.2025**
(21) Application number: 21796818.9
(22) Date of filing: 23.04.2021
(51) Int. Cl.: G01N 33/543, G01N 27/327, B01L 3/00

(54) **DETECTION DEVICE AND DETECTION METHOD**
DETEKTIONSVORRICHTUNG UND DETEKTIONSVERFAHREN
DISPOSITIF DE DÉTECTION ET PROCÉDÉ DE DÉTECTION

(30) Priority: 28.04.2020 JP 2020079367
(43) Date of publication of application: 08.03.2023
(73) Proprietor: Denka Company Limited, Tokyo 103-8338 (JP)
(72) Inventor: AKIYAMA, Yuto, Tokyo 103-8338 (JP); AOYAMA, Shuhei, Tokyo 103-8338 (JP)
(74) Representative: Schnappauf, Georg
(86) International application number: PCT/JP2021/016421
(87) International publication number: WO 2021/220956

(56) References cited:
- EP-A1- 3 470 842
- WO-A1-03/103835
- WO-A1-2009/031375
- WO-A1-2017/217406
- WO-A1-2019/117102
- WO-A1-2019/117102
- WO-A1-2021/185695
- WO-A2-2005/089082
- JP-A- 2005 532 151
- JP-A- 2008 096 235
- JP-A- 2008 096 235
- JP-A- 2009 204 339
- JP-A- 2010 085 333
- JP-A- 2017 032 416
- JP-A- H09 269 325
- US-A1- 2021 181 194
- NUMTHUAM SONTHAYA ET AL: "Synergistic effects of micro/nano modifications on electrodes for microfluidic electrochemical ELISA", SENSORS AND ACTUATORS B: CHEMICAL, vol. 156, no. 2, 1 August 2011 (2011-08-01), NL, pages 637 - 644, XP093105437, ISSN: 0925-4005, DOI: 10.1016/j.snb.2011.02.010
- KIM HYUNG JIN ET AL: "Advancing liquid front shape control in capillary filling of microchannel via arrangement of microposts for microfluidic biomedical Sensors", INTERNATIONAL JOURNAL OF PRECISION ENGINEERING AND MANUFACTURING, KOREAN SOCIETY FOR PRECISION ENGINEERING, SPRINGER, vol. 17, no. 1, 12 January 2016 (2016-01-12), pages 59 - 63, XP035720034, ISSN: 2234-7593, [retrieved on 20160112], DOI: 10.1007/S12541-016-0008-X

## Description

### TECHNICAL FIELD

The present invention relates to a detection device for detecting a substance to be detected in a liquid sample and a detection method.

### BACKGROUND ART

In recent years, Point of Care Test (POCT) reagents that use antigen-antibody reactions or the like to test for infectious diseases and pregnancy and to measure blood glucose levels and the like have attracted attention. It is possible for tests and measurements using POCT reagents to determine results in a short period of time. In addition, methods using POCT reagents are simple and POCT reagents are inexpensive. Since POCT reagents have these characteristics, POCT reagents are widely used in medical check-ups at the stage when symptoms are mild, regular check-ups, or the like. In addition, POCT reagents are also an important diagnostic tool in home health care, which is expected to increase in the future.

In examinations or diagnosis using an examination kit, which has a type of POCT reagent, a liquid sample such as blood is introduced into the examination kit and a specific substance to be detected included in the liquid sample is detected. The immunochromatography method is a commonly used method for detecting specific substances to be detected in liquid samples. In the immunochromatography method, a liquid sample is dropped onto a membrane carrier provided in the examination kit such that, in the process of the liquid sample moving over the membrane carrier, the substance to be detected in the liquid sample binds to a labeling substance. Furthermore, the substance to be detected binds specifically and selectively to the substance fixed in the examination kit (referred to below as the "detection substance"). The resulting change in color, weight, or the like occurring in the examination kit is detected. The detection substance may be referred to as a reagent. A microfluidic Point-Of-Care Assay using an integrated fluid sample test strip performing an ELISA or ELONA test is described in Patent Document 4 which is prior art under Art. 54(3) EPC.

Nitrocellulose membranes are often used as membrane carriers for moving liquid samples (refer to Patent Document 1 below). Nitrocellulose membranes have a large number of micropores with a diameter of approximately several µm and the liquid sample moves through these pores due to capillary force.

However, nitrocellulose membranes are derived from natural products and the flow velocity of the liquid sample through the membrane varies according to the membrane because the pore size and the manner in which the pores are connected to each other in the membrane are not uniform. When variations occur in the flow velocity, the time required to detect the substance to be detected also varies. As a result, there is a possibility that an incorrect judgment may be made in which the substance to be detected is not detected before the substance to be detected binds to the labeling substance or reagent.

To solve the problem described above, a method was devised for artificially creating micro-flow channels for a liquid sample (refer to Patent Documents 2 and 3 described below). By using this method, it is possible to produce a membrane carrier which has a uniform structure. As a result, it is possible to reduce the possibility of an incorrect judgment being made in which the substance to be detected is not detected before the substance to be detected binds to the labeling substance or reagent.

Synergistic effects of micro/nano modifications on electrodes for microfluidic electrochemical ELISA are disclosed in Non-Patent Document 1. An electrochemical measuring microchip which is said to have high detection sensitivity is described in Patent Document 5. Membrane carriers for liquid sample test kits, liquid sample test kits and methods for producing liquid sample test kits are disclosed in Patent Documents 6 and 7.

### RELATED DOCUMENT

### PATENT DOCUMENTS

[Patent Document 1] Japanese Unexamined Patent Application, First Publication No. 2014-062820
[Patent Document 2] Japanese Patent No. 4597664
[Patent Document 3] Published Japanese Translation No. 2012-524894 of the PCT International Publication
[Patent Document 4] Published International Application WO 2021/185695 A1
[Patent Document 5] Japanese Patent Application No. 2008-96235
[Patent Document 6] Published European Patent Application No. 3 470 842 A1
[Patent Document 7] Published International Application WO 2019/117102 A1

### NON-PATENT DOCUMENT

[Non-Patent Document 1] S. Numthuam et al., Sensors and Actuators B 156(2011), 637-644

### SUMMARY OF THE INVENTION

### TECHNICAL PROBLEM

In the immunochromatography method, a liquid sample is dropped onto a membrane carrier provided in an examination kit such that, in the process of the liquid sample moving over the membrane carrier, the substance to be detected in the liquid sample binds to the labeling substance. Furthermore, the substance to be detected binds specifically and selectively to a detection substance fixed in the examination kit. The resulting change in color, weight, or the like occurring in the examination kit is detected. There is a well-known method (color change detection method) for detecting a substance to be detected, in which, for a substance to be detected which is bound to a labeling substance such as colored latex particles, fluorescent particles, or metal colloidal particles, an optical measuring instrument such as an absorbance meter is used to detect color changes in a detection zone caused by binding to a reagent fixed in the detection zone. In addition, there is also a method (electrochemical immunochromatography method) in which the concentration of a biomarker is detected by being converted into an electrochemically active substance concentration.

In the electrochemical immunochromatography method, there are issues in that the deployment of a plurality of types of solutions, such as reaction solutions, cleaning solutions, and secondary reaction solutions is necessary, the time and effort required for the use thereof is increased, and the detection time is increased and these have been obstacles to the widespread use of examination kits using the electrochemical immunochromatography method. That is, for examination kits using the electrochemical immunochromatography method, there was a demand for a POCT reagent (examination kit) that is capable of determining results in a short period of time, that has an easy use method, and that is inexpensive. Even in a case of using the color change detection method, there were similar issues with methods for which the deployment of a plurality of types of solutions was necessary.

The present invention was created in view of the circumstances described above and has an object of providing a technique that makes it possible to save time and effort during use and shorten detection time in a case where a plurality of types of solutions such as a reaction solution, a cleaning solution, and a secondary reaction solution are deployed in an examination kit using the immunochromatography method.

### SOLUTION TO PROBLEM

The present invention is defined by the claims.

An examination device (also called an "examination kit") of the present invention includes (i) a flow channel provided on a substrate formed of resin and transporting a liquid sample from one end side to the other end side, (ii) a solid-phase part provided on the other end side of the flow channel, in which an antibody is set to a solid-phase, (iii) a detection unit provided with an electrode portion to detect a reaction of the liquid sample with respect to the antibody, (iv) a fine uneven structure having a plurality of protrusions formed integrally with the flow channel, in which the fine uneven structure has a first uneven portion in which the plurality of protrusions are provided relatively loosely, and a second uneven portion in which the plurality of protrusions are provided relatively densely, and the first uneven portion and second uneven portion are provided further toward the one end side of the flow channel than the solid-phase part, and (v) an introduction portion for introducing the liquid sample into the flow channel, wherein the liquid sample is formed of a plurality of types of solutions, and the introduction portion is provided at a plurality of locations according to the plurality of types of solutions; the detection unit is provided further toward the other end side of the flow channel than the solid-phase part; and wherein the first uneven portion and the second uneven portion are adjacent, a ratio (P1/P2) of a pitch (P1) between the protrusions in the first uneven portion and a pitch (P2) between the protrusions in the second uneven portion is 1.1 or more and 5 or less.

The examination device of the present invention is a detection device for detecting a substance to be detected in a liquid sample.

A detection method of the present invention detects a reaction of a liquid sample with respect to an antibody using the detection device described above.

### ADVANTAGEOUS EFFECTS OF INVENTION

According to the present invention, it is possible to provide a technique that makes it possible to save time and effort during use and shorten detection time in a case where a plurality of types of solutions such as a reaction solution, a cleaning solution, and a secondary reaction solution are deployed in an examination kit using the immunochromatography method.

### BRIEF DESCRIPTION OF THE DRAWINGS

Fig. 1 is a top surface view of an examination kit of a first embodiment.
Fig. 2 is a top surface view of a membrane carrier of the first embodiment.
Fig. 3 is a diagram showing a fine structure and protrusions of the first embodiment.
Fig. 4 is a perspective view of a protrusion of the first embodiment.
Fig. 5 is an enlarged diagram showing boundaries of each region of the fine structure of the first embodiment.
Fig. 6 is a chart illustrating an example of an examination method using the examination kit of the first embodiment.
Fig. 7 is a top surface view of a membrane carrier of a second embodiment.
Fig. 8 is an enlarged diagram showing boundaries of each region of a fine structure of the second embodiment.
Fig. 9 is a diagram showing an example of a backflow-preventing structure for the solution in the fine structure of the second embodiment.
Fig. 10 is a diagram showing a photograph of a test specimen of Example 1.
Fig. 11 is a diagram showing the test specimen in images taken 30, 140, and 310 seconds after the start of the test in Example 1.
Fig. 12 is a diagram showing a graph plotting the elapsed time and the RGB component ratio of the measurement points in Example 1.
Fig. 13 is a diagram showing a graph showing the results of calculating the mixing ratio of each solution from the results of analyzing the data in Fig. 12 of Example 1.
Fig. 14 is a diagram showing a graph showing the results of calculating the mixing ratio of each solution in Comparative Example 2 under the condition that nitrocellulose was used instead of an imprinted sheet.
Fig. 15 is a diagram showing the configuration of the test specimen in Example 2.
Fig. 16 is a diagram showing a graph of the test results of Example 2.
Fig. 17 is a diagram showing a graph of the fluorescence intensity of the AB portion and BG portion of Example 2.
Fig. 18 is an example of a fluorescence photograph of Example 2.
Fig. 19 is a diagram showing an arrangement example of electrode portions in the two-electrode system of the first embodiment.
Fig. 20 is a diagram showing an arrangement example of electrode portions in the three-electrode system of the first embodiment.
Fig. 21 is a diagram showing the configuration of the test specimen of Example 3.
Fig. 22 is a diagram showing a graph of the test results of Example 3.
Fig. 23 is a chart showing the timing of solution drops in Example 4.
Fig. 24 is a diagram showing a graph of the test results of Example 4.

### DESCRIPTION OF EMBODIMENTS

### <First Embodiment>

A description will be given below of an embodiment of the present invention.

### <Overview of Examination kit>

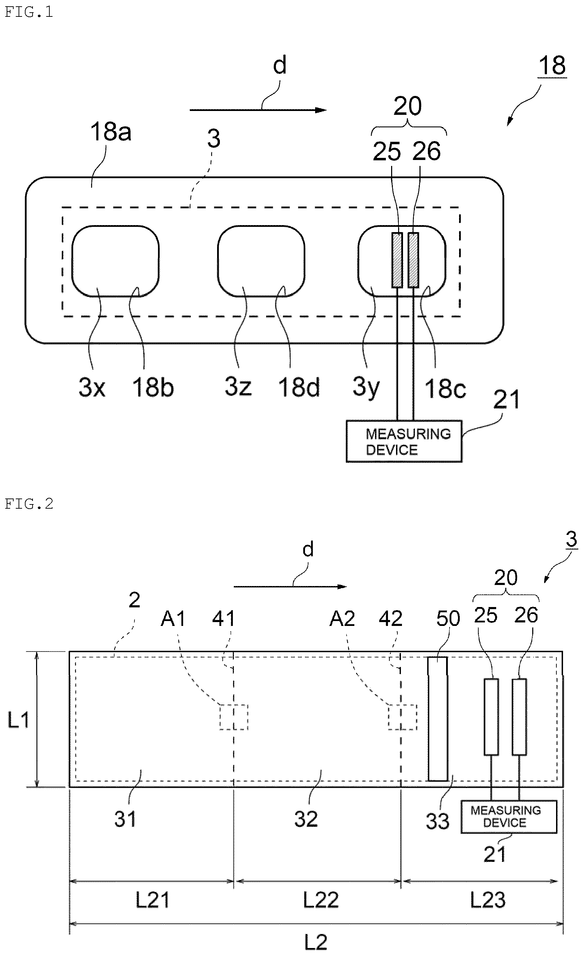

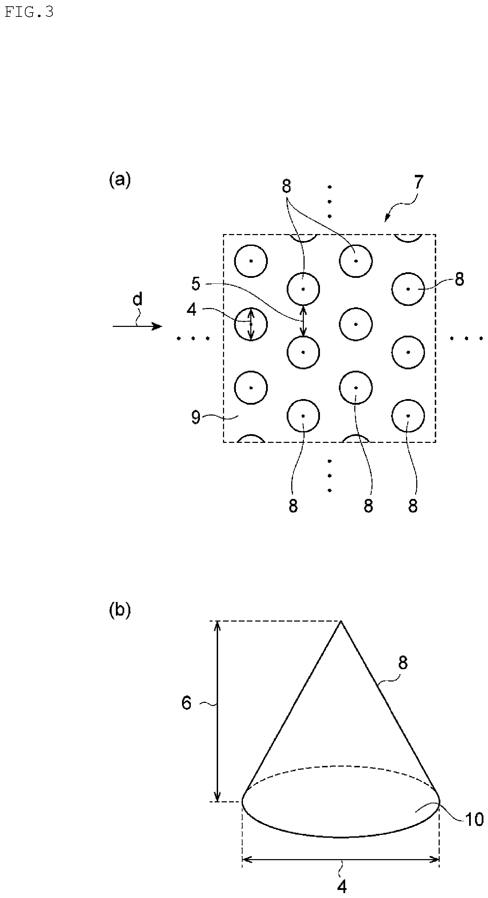

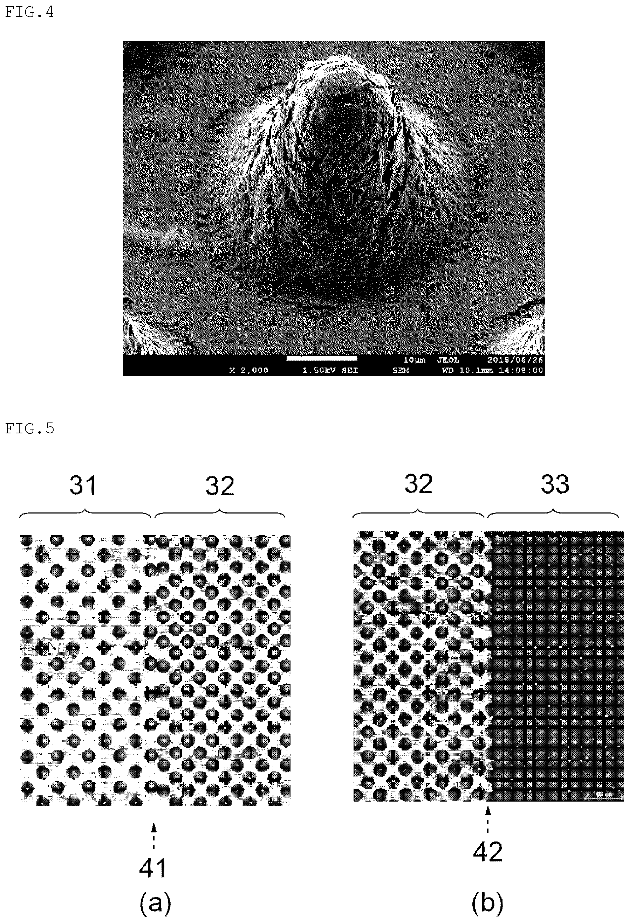

Fig. 1 is a plan view of an examination kit 18 according to the present embodiment. Fig. 2 shows a plan view schematically showing a membrane carrier 3. Fig. 3 shows a fine structure of the membrane carrier 3 (also referred to as a "fine uneven structure") and a protrusion 8 forming the above. Fig. 4 shows a perspective view (SEM image) of the protrusion 8.

The examination kit 18 has the function of detecting the substance to be detected in the liquid sample.

As described in detail below, the examination kit 18 is a type of POCT reagent. A liquid sample, such as blood, is introduced into the examination kit 18 and a specific substance to be detected included in the liquid sample is detected. The immunochromatography method is applied as the method for detecting a specific substance to be detected from the liquid sample.

In the present embodiment, a description will be given of the examination kit 18 which is configured using the electrochemical immunochromatography method, in which the concentration of a biomarker is converted to an electrochemically active substance concentration for detection.

As described above, in the electrochemical immunochromatography method, the deployment of a plurality of types of solutions (liquid samples) such as a reaction solution, a cleaning solution, and a secondary reaction solution, is necessary, and, generally, the time and effort required for use is increased and the detection time is increased. In the present embodiment, a flow channel 2 of the membrane carrier 3 is divided into a plurality of areas (here, three areas of first to third fine structure regions 31 to 33) and the flow velocity of the solution is controlled to be different in each area. As the structure for making the flow velocity different, the fine structures formed in the membrane carrier 3, that is, structures that cause a capillary action that determines the speed at which the solution is transported, were set for each area.

The details are described below.

### <Details of Examination kit 18>

As shown in Fig. 1, the examination kit 18 is provided with the membrane carrier 3 and a housing 18a that accommodates the membrane carrier 3. For this figure, a description will be given with the direction from upstream on the left to downstream on the right in the figure as a travel direction d of the solution (also called "flow channel direction") .

The surface of the membrane carrier 3 has, in order from the left side in the figure, a cleaning solution zone 3x, where a cleaning solution is dropped, a droplet zone 3z, where the liquid sample is dropped, and a detection zone 3y for detecting the substance to be detected in the liquid sample. Although not shown here, an absorbent pad for absorbing excess solution is provided on the downstream side from the membrane carrier 3 (on the right side in the figure).

The cleaning solution zone 3x is exposed at a first opening 18b of the housing 18a. The droplet zone 3z is exposed at a third opening 18d of the housing 18a. The detection zone 3y is exposed at a second opening 18c of the housing 18a. The cleaning solution may be dropped in the droplet zone 3z, in which case the first opening 18b may be omitted. In a case of a plurality of types of solutions, introduction ports (openings) are provided according to those solutions. That is, the introduction ports are provided according to what kind of solution is to be moved, at what timing, and at what speed. A plurality of solutions may be dropped at a particular introduction port and the timing thereof may be the same or different.

In the detection zone 3y, an electrode portion 20 is provided for detection using the electrochemical detection method. The electrode portion 20 is, for example, two electrodes (a two-electrode system) formed of a working electrode 25 on the upstream side in the travel direction d and a counter electrode 26 on the downstream side thereof. The electrode portion 20 may be a three-electrode system having a reference electrode 27, as described below. A measuring device 21 is connected to the electrode portion 20. The measuring device 21 may be a general measuring device or may be configured as a device in which a predetermined application is introduced in a mobile terminal such as a smart phone.

### <Details of Membrane Carrier 3>

As shown in Fig. 2, the membrane carrier 3 is provided with at least one flow channel 2 for transporting a liquid sample. As shown in Fig. 3, a fine structure 7 is provided on the bottom surface of the flow channel 2. In the present embodiment, the fine structure 7 is provided over the entire surface of the membrane carrier 3 and the entire surface of the membrane carrier 3 functions as the flow channel 2 for the liquid sample.

Fig. 3(a) is a top surface view of the fine structure 7 and Fig. 3(b) is a perspective view of the protrusion 8 forming the fine structure. The fine structure 7 is the totality of the protrusions 8. In other words, the membrane carrier 3 is provided with a flat portion 9 corresponding to the bottom surface of the flow channel 2 for the liquid sample and a plurality of the protrusions 8 protruding from the flat portion 9.

Due to capillary action, the space between the plurality of protrusions 8 functions as the flow channel 2 that transports the liquid sample along the surface of the membrane carrier 3. In other words, due to the capillary action, gaps in the fine structure 7 function as the flow channel 2 for transporting the liquid sample along the surface of the membrane carrier 3. The plurality of protrusions 8 are formed in rows in regular alignment on the surface of the membrane carrier 3 in a regular or translationally symmetric manner, such as in a lattice arrangement (for example, a diamond-shaped lattice arrangement or a regular lattice arrangement).

The protrusions 8 are, for example, cones and, here, the protrusions 8 have a circular cone shape, as shown in Fig. 3(b) and Fig. 4. Moreover, the protrusions 8 may be a pyramid or may be a shape in which the upper part of the cone is cut off (a truncated cone). In any case, it is sufficient if the fine structure 7 formed of the protrusions 8 generates capillary action and transports the liquid sample.

The fine structure 7 generates capillary action. Due to the capillary action of the fine structure 7, the liquid sample is transported through the fine structure 7 from the cleaning solution zone 3x or the droplet zone 3z on the left side of the figure to the detection zone 3y (along the travel direction d in Fig. 2).

In the present embodiment, as shown in Fig. 2, the membrane carrier 3 is divided into three areas from the left side which are the first fine structure region 31 (first uneven portion), the second fine structure region 32 (second uneven portion), and the third fine structure region 33 (third uneven portion). The first fine structure region 31, the second fine structure region 32, and the third fine structure region 33 have different fine structures 7 and, as a result, each area transports the solution at a different speed.

The speed at which the solution is transported is understood from Poiseuille's equation, which describes the flow between parallel plates. For example, in the fine structure 7 that generates the capillary action phenomenon, for example, in a structure in which a plurality of the protrusions 8 are arranged, the narrower a distance 5 between the protrusions 8, the greater the transport speed of the solution. That is, by appropriately setting the looseness and density of the fine structure (the arrangement of the protrusions 8 shown in Fig. 3), it is possible to control the speed for each area.

Fig. 5 shows views of the fine structure 7 from the top surface. Fig. 5(a) shows the region of the boundary (a first boundary 41) between the first fine structure region 31 and the second fine structure region 32. Fig. 5(b) shows the region of the boundary (a second boundary 42) between the second fine structure region 32 and the third fine structure region 33. In all regions, the protrusions 8 are provided in the same shape and size. In Fig. 5, the protrusions 8 are circular cones with a bottom surface diameter of 30 µm and a height of 30 µm. As shown in the figures, the arrangement (degree of looseness and density) of the protrusions 8 is loosest in the first fine structure region 31 on the left side of the figure and densest in the third fine structure region 33 on the right side. That is, the distance 5 between the protrusions 8 in the first fine structure region 31 is the widest and the distance 5 between the protrusions 8 in the third fine structure region 33 is the narrowest. In the example shown in the figures, the distance 5 between the protrusions 8 of the first fine structure region 31 is 25 µm, the distance 5 between the protrusions 8 of the second fine structure region 32 is 15 µm, and the distance between the protrusions 8 of the third fine structure region 33 is 2 µm.

When the substance to be detected in the liquid sample reaches the detection zone 3y, it is detected as a current value by the measuring device 21 through the electrode portion 20 (the working electrode 25 and the counter electrode 26) provided in the detection zone 3y. That is, a potential difference is applied between the working electrode 25 and the counter electrode 26 of the electrode portion 20 and the oxidation current is measured by the measuring device 21. In a case where the color change detection method is used, the substance to be detected is detected according to the color change of the detection zone 3y.

### <Material of Membrane Carrier 3>

The membrane carrier 3 including the fine structure 7 (a plurality of the protrusions 8) is formed of, for example, thermoplastic plastic. That is, it is possible to produce the membrane carrier 3 having the fine structure 7 by processing a membrane base material formed of thermoplastic plastic by thermal imprinting. The thermoplastic plastic forming the membrane carrier 3 may be, for example, at least one selected from the group consisting of polyester-based resins, polyolefin-based resins, polystyrene-based resins, polycarbonate-based resins, fluorinated resins, and acrylic-based resins. Specific thermoplastic plastics may be, for example, at least one formed of polyethylene terephthalate (PET), cyclo-olefin polymers (COP), polypropylene (PP), polystyrene (PS), polycarbonate (PC), polyvinylidene fluoride (PVDF), and polymethyl methacrylate (PMMA).

The glass transition point Tg or melting point Tm of the thermoplastic plastics described above may be 80°C to 180°C. The storage modulus of the thermoplastic plastic at a temperature 20°C higher than the glass transition point Tg may be 1.0 Pa or more and 1.0 x 10⁷ Pa or less. The storage modulus of the thermoplastic plastic at a temperature 20°C higher than the melting point Tm may be 1.0 Pa or more and 1.0 x 10⁷ Pa or less.

In a case where the glass transition or melting of the thermoplastic plastic occurs at a temperature of less than 80°C and the storage modulus of the thermoplastic plastic at a temperature 20°C higher than the glass transition point or melting point is 1.0 x 10⁷ Pa or less, it is difficult in practice to use the thermoplastic plastic as a solid at room temperature and difficult to produce a membrane carrier by thermal imprinting.

In a case where the glass transition or melting of the thermoplastic plastic occurs at a temperature higher than 180°C, the molding temperature during thermal imprinting becomes high and the productivity of the membrane carrier decreases. In other words, in a case where the temperature required to soften the thermoplastic plastic during thermal imprinting is higher than 180°C, the productivity of the membrane carrier is reduced.

In a case where the storage modulus of the thermoplastic plastic at a temperature 20°C higher than the glass transition point or melting point is 1.0 x 10⁷ Pa or less, it is possible to keep the molding pressure required to produce the fine structure small and the production efficiency is improved because it is possible to carry out production under relatively mild conditions.

It is possible to form the protrusions 8 of the cones (here, circular cones) by thermal imprinting using a mold. In a case of forming a cone using a mold, in comparison with forming a grooved flow channel (line-and-space structure) using a mold, the volume of metal to be scraped from the surface of the metal member during mold production is greatly reduced and the processing cost of the mold is reduced. In contrast, production of a mold for forming a line-and-space structure requires a large volume of metal to be scraped from the metal member.

In addition, the upper part of the cone is thinner than the bottom surface of the cone. Accordingly, in a case where a mold is used to form a cone, in comparison with a case where a column having the same bottom surface as the cone is formed using a mold, the volume of metal that is scraped from the surface of the metal member during the production of the mold is greatly reduced and the processing cost of the mold is reduced.

Furthermore, the porosity of the fine structure with regularly aligned cones is greater than the porosity of a line-and-space structure. In addition, the porosity of the fine structure with regularly aligned cones is greater than the porosity of a structure with a plurality of regularly aligned columns having the same bottom surface as the cone. Therefore, according to the fine structure with regularly aligned cones, it is possible to increase the flow velocity of the liquid sample, which is advantageous for the detection of the substance to be detected.

### <Shape and Dimensions of Membrane Carrier 3>

As described above, it is possible to freely select the shape of a bottom surface 10 of the cone (protrusion 8), which may be a circular cone as shown in Fig. 3(b) and Fig. 4, or a pyramid (such as a square pyramid or hexagonal pyramid). For ease of processing the mold and to suppress processing costs, it is desirable for the bottom surface 10 of the cones (protrusions 8) to be circular or polygonal (for example, a square, a rhombus, a rectangle, a triangle, a hexagon, or the like).

A diameter 4 of the bottom surface 10 of the protrusion 8 is, for example, 10 µm to 1000 µm. In a case where the diameter 4 of the bottom surface 10 of the protrusion 8 is smaller than 10 µm, the fine processing cost of the mold is increased and it is also difficult to uniformly produce numerous fine structures 7 on the surface of the membrane carrier 3, which has a large area. Accordingly, the fine structure 7 that is excessively small is not suitable for practical use. In a case where the diameter 4 of the bottom surface 10 of the fine structure 7 is smaller than 10 µm, the capillary force, which is necessary to move the liquid sample, tends to weaken. In a case where the diameter 4 of the bottom surface 10 of the fine structure 7 is larger than 1000 µm, the volume of metal to be scraped from the metal member during production of the mold becomes larger and the production cost of the mold and membrane carrier 3 is increased. In addition, in a case where the diameter 4 of the bottom surface 10 of the fine structure 7 is larger than 1000 µm, the area of the flow channel 2 in the membrane carrier 3 is also increased, which greatly increases the size of the examination kit 18, which is disadvantageous for the transportation of the examination kit 18 itself. In a case where the protrusion 8 (the fine structure 7) is a circular cone, the diameter 4 of the bottom surface 10 of the protrusion 8 may be the diameter 4 of the bottom surface 10 (circle) of the circular cone.

The height 6 of the protrusion 8 is, for example, 10 µm to 500 µm. In a case where the height 6 of the protrusion 8 is lower than 10 µm, the capillary force, which is necessary to move the liquid sample, tends to weaken. In a case where the height 6 of the protrusion 8 is higher than 500 µm, it is difficult to completely fill the thermoplastic plastic into the recesses of the mold (the depressions corresponding to the shapes of the protrusions 8 of the fine structure 7) during thermal imprinting.

The overall shape of the membrane carrier 3 is not particularly limited, but may be, for example, polygonal, such as a square, circular, or oval. In a case where the membrane carrier 3 is a rectangle, a length L1 of the membrane carrier 3 may be, for example, 2 mm to 100 mm, and a width L2 of the membrane carrier 3 may be, for example, 3 mm to 100 mm. In addition, widths L21 to L23 of the first to third fine structure regions 31 to 33 may each be, for example, 1 mm to 50 mm. The thickness of the membrane carrier 3 excluding the height 6 of the fine structure 7 (that is, the protrusions 8) may be, for example, 0.1 mm to 10 mm.

An aspect ratio Lv/Lh of the protrusion 8 may be 1/10 or more and 2/1 or less. In a case where the aspect ratio Lv/Lh is smaller than 1/10, the contact area between the liquid sample and the flow channel 2 is small and the capillary force decreases, which tends to make it difficult to move the liquid sample. In a case where the aspect ratio Lv/Lh is larger than 2/1, the productivity of the membrane carrier 3 by thermal imprinting decreases. In a case where the protrusion 8 is a cone (more specifically, a circular cone), as in the present embodiment, a length Lh of the protrusion 8 in the horizontal direction may be the diameter 4 of the bottom surface 10 of the protrusion 8. In addition, a length Lv of the protrusion 8 in the orthogonal direction may be the height 6 of the protrusion 8 from the flat portion 9 of the membrane carrier 3.

A ratio D2/D1 of the diameter 4 (D1) of the bottom surface of the protrusion 8 and a distance (D2) between the nearest centers of the protrusions 8 may be greater than 1 and 5 or less. The ratio D2/D1 may not be 1 or less. In a case where the ratio D2/D1 is greater than 5, the contact area between the liquid sample and the flow channel 2 decreases, the capillary force decreases, and the liquid sample tends to be difficult to move. In a case where the protrusion 8 is a circular cone, as in the present embodiment, the diameter 4 (D1) of the bottom surface 10 of the protrusion 8 may be the diameter of the bottom surface of the circular cone and a distance D2 between the nearest centers may be the distance between the vertices of a pair of adjacent protrusions 8 (circular cones) . The diameter 4 (D1) of the bottom surface 10 of the protrusions 8 may match the length Lh of the protrusion 8 in the horizontal direction described above. Accordingly, the aspect ratio Lv/Lh may be expressed as Lv/D1.

In addition, in a case where the pitches (distance between vertices) between the protrusions 8 of the fine uneven structures are compared between adjacent zones, the ratio (P1/P2) of the pitch P1 of the zone of the fine uneven structure configured relatively loosely to the pitch P2 of the zone of the fine uneven structure configured relatively densely is 1.1 or more and 5 or less. Here, in a case where the pitch between the protrusions 8 in the first fine structure region 31 is P11, the pitch between the protrusions 8 in the second fine structure region 32 is P21, and the pitch between the protrusions 8 in the third fine structure region 33 is P23, the ratio (P11/P21) is 1.1 or more and 5 or less and the ratio (P21/P23) is 1.1 or more and 5 or less. The ratio (P1/P2) is set according to the speed at which the solution is to be moved. For the lower limit of the ratio (P1/P2), in a case of being small, there will be no difference in speed between zones and the significance of providing a difference in the degree of looseness and density of the fine uneven structure will decrease. From this viewpoint, the ratio (P1/P2) is preferably 1.2 or higher and more preferably 1.3 or higher. For the upper limit, in a case of being excessively large, the difference in the solution movement speed between zones becomes excessively large, making it difficult to adjust the speed throughout the examination kit 18. From this viewpoint, an upper limit of 4 or less is preferable and 3 or less is more preferable.

### <Arrangement of Electrode Portion>

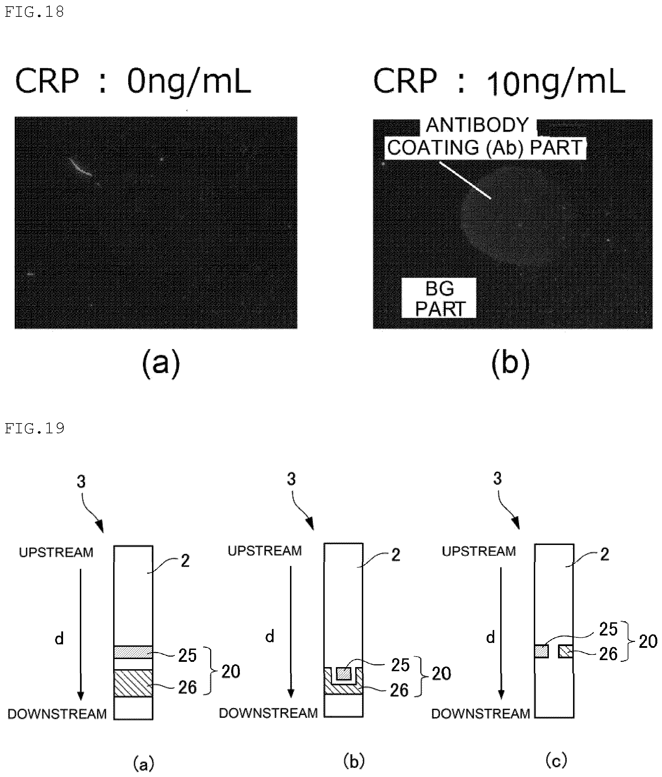

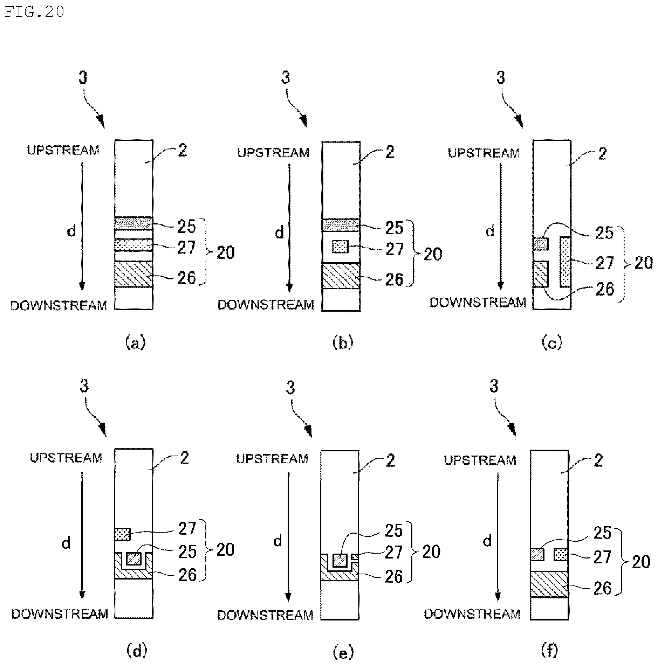

Referring to Fig. 19 and Fig. 20, a description will be given of arrangement examples of the electrode portion 20.

Fig. 19(a) to Fig. 19(c) show arrangement examples of the working electrode 25 and the counter electrode 26 in a case where the electrode portion 20 is a two-electrode system. The working electrode 25 and the counter electrode 26 are provided to be separated from each other. Here, the working electrode 25 is provided at the same position as the counter electrode 26 with respect to the travel direction d or on the upstream side from the counter electrode 26. The working electrode 25 may be formed, for example, as a comb-shaped electrode.

In the arrangement example shown in Fig. 19(a), the working electrode 25 is provided over the entire width direction of the flow channel 2. The counter electrode 26 is provided over the entire width direction of the flow channel 2 in a region that is separated by a predetermined distance to the downstream side from the working electrode 25. The working electrode 25 and counter electrode 26 are rectangular in a top surface view; however, various shapes such as elliptical or semi-circular are possible without being limited to the above shape. In addition, the working electrode 25 and counter electrode 26 are provided so as to block the width direction of the flow channel 2, but, without being limited thereto, may be provided only in a partial region with respect to the width direction, as shown in Fig. 1 and Fig. 2. The width of both or either of the working electrode 25 or counter electrode 26 may be shortened.

In the arrangement example shown in Fig. 19(b), the counter electrode 26 is provided in a "U" shape with the upstream side concave in the top surface view. Furthermore, the working electrode 25 is provided in a rectangular shape in the concave shape region of the counter electrode 26. The most upstream side position of the working electrode 25 is the same position as the upstream side position of the counter electrode 26.

In the arrangement example shown in Fig. 19 (c), the rectangular working electrode 25 and the counter electrode 26 are each provided symmetrically in the width direction.

Fig. 20(a) to Fig. 20(f) show arrangement examples of the working electrode 25, the counter electrode 26, and the reference electrode 27 in a case where the electrode portion 20 is a three-electrode system.

In the arrangement example shown in Fig. 20(a), from the upstream side to the downstream side, the working electrode 25, the reference electrode 27, and the counter electrode 26 are provided in a row over the entire width direction of the flow channel 2. This may be said to be the arrangement shown in Fig. 19(a), in which the reference electrode 27 is arranged between the working electrode 25 and the counter electrode 26.

In the arrangement example shown in Fig. 20(b), there is a configuration in which, in the arrangement shown in Fig. 20(a), the width of the reference electrode 27 is shortened to be provided as a rectangle in the center of the flow channel 2 in the width direction.

In the arrangement example shown in Fig. 20(c), the working electrode 25 and counter electrode 26 are provided from the upstream side on the left side of the flow channel 2 in the figure and the reference electrode 27 is provided on the right side of the flow channel 2. The reference electrode 27 is provided to be elongated in the travel direction d from a position on the upstream side of the working electrode 25 to a position on the downstream side of the counter electrode 26.

In the arrangement example shown in Fig. 20(d), the working electrode 25 and the counter electrode 26 are similar to the arrangement shown in Fig. 19(b), with the reference electrode 27 being further provided at a position on the left side of the figure upstream from the working electrode 25.

In the arrangement example shown in Fig. 20(e), the concave right-side edge of the counter electrode 26 arranged as shown in Fig. 19(b) is shortened to the downstream side and the reference electrode 27 is provided in the shortened region.

In the arrangement example shown in Fig. 20(f), the counter electrode 26 is provided over the entire width direction of the flow channel 2. Furthermore, the working electrode 25 is provided on the left side of the flow channel 2 and the reference electrode 27 is provided on the right side, so as to be symmetrical.

### <Configuration of Electrode Portion>

The electrode portion 20 (the working electrode 25 and the counter electrode 26 in the case of a two-electrode system and the reference electrode 27 in the case of three electrodes) may be formed by a conductor substance being provided on the protrusions 8 of the fine structure 7. The conductor substance is not particularly limited and examples thereof include gold, silver, platinum, palladium, carbon, graphene, carbon nanotubes (CNTs), composite materials thereof, and the like. The reference electrode 27 is not particularly limited and examples thereof include an Ag/AgCl electrode.

The conductor substance of the protrusion 8 is, for example, a conductor film formed using at least one of sputtering, vacuum evaporation, laser ablation, and chemical vapor deposition (CVD), or a printed layer formed of a paste (ink) including conductor particles by a method such as ink jet printing or screen printing. The working electrode 25 may be surface modified with thiols or the like for antibody fixation.

In such a case, a maximum peak height Rp of the roughness curve of the electrode portion 20 is 0.005 µm or more and 10 µm or less and an average length RSm of the roughness curve elements is 0.01 µm or more and 15 µm or less. Setting such a surface roughness makes it possible to generate favorable capillary force and to increase the amount of signal obtained due to the increased surface area of the electrode portion 20. It is possible to calculate the surface roughness by analyzing SEM images as shown in Fig. 4.

### <Method for Manufacturing Examination Kit 18>

The method for manufacturing the examination kit 18 obtains the examination kit 18 by the following steps.

### Step 1...Thermal imprinting step

There is provided a step of producing the membrane carrier 3 having a fine structure (plurality of protrusions 8) and corresponding to the shape of the recesses by applying the surface of a mold in which a plurality of recesses are formed to a membrane base material made of thermoplastic plastic and heating the base material.

The method for manufacturing the examination kit 18 is further provided with a step of fixing a reagent or a labeling substance to the detection zone 3y of the surface of the membrane carrier 3 with the fine structure 7, more specifically, to a solid-phase part 50 of the third fine structure region 33.

The fine production method of the mold used in the thermal imprinting step may be, for example, etching, photolithography, machine cutting, laser machining, or the like. It is possible to select a fine production method suitable for the processing size and processing range.

Before performing the thermal imprinting, it is desirable to perform a mold release treatment. In the mold release treatment, for example, a monolayer may be produced on the mold surface to reduce the surface energy. As a result, the membrane carrier 3 formed of thermoplastic plastic is easily detached from the surface of the mold after the thermal imprinting.

The thermal imprinting method may be either a flat press method or a roll method. In the flat press method, the mold is overlaid with base materials formed of thermoplastic plastic between parallel upper and lower stages which face each other, and these are placed between the stages. The mold and base materials are then heated and pressed through the stages. The flat press method is superior in the point of providing favorable molding accuracy. The roll method uses a heated roll mold and molding is performed by the squeezing pressure between the rolls. The roll method has excellent productivity.

Conditions such as the molding temperature, molding pressure, and transfer time when performing thermal imprinting may be selected according to the size of the fine processing, the shape of the fine structure (the protrusions 8), the size of the processing range, and the like. For example, in the case of the flat press method, the molding temperature may be 20°C to 50°C higher than the glass transition point Tg or 20°C to 50°C higher than the melting point Tm. The molding pressure may be 1 MPa to 10 MPa. The transfer time (time to hold the mold and base materials while applying pressure) may be 3 to 10 minutes. Thermal imprinting under the above conditions makes accurate transfer of the fine structure of the mold to the base material surface easy.

Depending on the type of thermoplastic plastic forming the membrane carrier 3 and the type of reagent (detection substance), it may be difficult to fix the reagent (detection substance) to the solid-phase part 50 of the membrane carrier 3. In such a case, by applying an appropriate surface treatment to the detection zone 3y only in advance, the reagent (detection substance) is easily fixed to the detection zone 3y (that is, the solid-phase part 50) of the membrane carrier 3.

The surface treatment method of the detection zone 3y is not limited at all and may be, for example, various plasma treatments, UV treatments, UV/ozone treatments, or various methods such as surface modification using 3-aminopropyltriethoxysilane or glutaraldehyde.

The reagent (detection substance) fixed in the detection zone 3y is an antibody. For example, in Fig. 2, the antibody is fixed on the solid-phase part 50 of the third fine structure region 33. The solid-phase part 50 is provided on the upstream side of the electrode portion 20 in the travel direction d of the solution.

The antibody is a substance that causes an antigen-antibody reaction with the substance to be detected. The antibody may be a polyclonal antibody or a monoclonal antibody. The substance to be detected is not limited at all and may be any substance capable of causing an antigen-antibody reaction with the antibody, such as various pathogens and various clinical markers. Specifically, the substance to be detected may be, for example, virus antibodies for the influenza virus, norovirus, adenovirus, RS virus, HAV, HBs, HIV, and the like. The substance to be detected may be a bacterial antigen for MRSA, group A streptococci, group B streptococci, Legionella spp. , or the like, or toxins produced by bacteria or the like. The substance to be detected may be mycoplasma, Chlamydia trachomatis, or a hormone such as human chorionic gonadotropin. The substance to be detected may be a C-reactive protein, myoglobin, cardiac troponin, various tumor markers, pesticides, environmental hormones, and the like. In particular, in a case where there is an urgent need to detect substances to be detected such as the influenza virus, norovirus, C-reactive protein, myoglobin, and cardiac troponin and to take therapeutic measures for diseases caused by these substances, the usefulness of the examination kit 18 according to the present embodiment is particularly great. The substance to be detected may be an antigen able to induce an immune reaction by itself. The substance to be detected may be a hapten that is not able to induce an immune reaction by itself, but is able to bind to an antibody by an antigen-antibody reaction with the antibody.

### <Examination Method Using Examination kit 18>

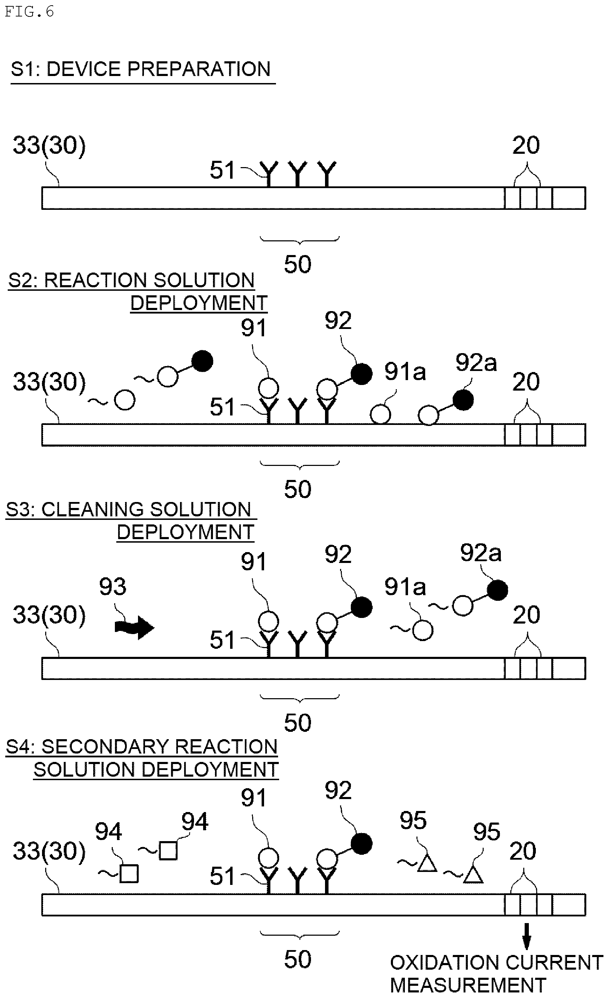

Referring to the chart diagram shown in Fig. 6 and to Fig. 1 to Fig. 5 described above, a description will be given of the examination method using the examination kit 18. In Fig. 6, the fine structure 30 is shown with a focus on the third fine structure region 33.

### S1: Device preparation step

First, the examination kit 18 and the solutions to be used (a reaction solution, a cleaning solution, and a secondary reaction solution) are prepared. As described above, an antibody 51 is fixed in the solid-phase part 50 of the third fine structure region 33.

### S2: Reaction solution deployment step

When the reaction solution is dropped from the droplet zone 3z into the second fine structure region 32, the reaction solution moves to the third fine structure region 33 due to the capillary action of the fine structure 7.

A detection target 91 and a detection target (labeling body) 92 in the reaction solution are fixed by reacting with the antibody 51. Excess reaction solution is absorbed by a water-absorbing pad; however, some of a detection target 91a and detection target (labeling body) 92a are not fixed and remain on the third fine structure region 33.

### S3: Cleaning solution deployment step

Subsequently, a cleaning solution 93 is dropped from the cleaning solution zone 3x to clean the detection target 91a and the detection target (labeling body) 92a that are not fixed to the solid-phase part 50 and remain on the third fine structure region 33. The detection target (labeling body) 92a has an alkaline phosphatase (ALP) labeling body.

### S4: Secondary reaction solution deployment step

After cleaning, a secondary reaction solution (for example, p-aminophenyl phosphate 94) dropped into the second fine structure region 32 moves to the third fine structure region 33 due to the capillary action of the fine structure 7. The p-aminophenyl phosphate 94 reacts with the detection target (labeling body) 92 fixed on the antibody 51 to produce an electrically active substance (here, p-aminophenol 95). This substance correlates (is proportional) to the amount (concentration) of the detection target (labeling body) 92 fixed on the antibody 51. Accordingly, it is possible to accurately and stably measure the concentration of the target to be measured by the value of the oxidation current measured at the electrode portion 20.

According to the present embodiment, in the examination kit 18 applying the immunochromatography method, it is possible to set the speed at which the solution is moved (speed due to the capillary action) in the flow channel 2 of the fine structure 7 of the membrane carrier 3 to be different in a plurality of regions. As a result, even in a case where it is necessary to deploy a plurality of types of solutions such as a reaction solution, a cleaning solution, and a secondary reaction solution, it is possible to adjust the timing of the deployment of these solutions according to the mode of use. Accordingly, it is possible to eliminate the time and effort for timing adjustments and the like, which would normally be necessary, and it is possible to carry out stable and appropriate examination. Specifically, using a specific jig or the like, it is possible to apply drops simultaneously to a plurality of different locations, in consideration of the timing at which each solution is deployed. That is, it is possible to deploy each solution in only one operation.

### <Second Embodiment>

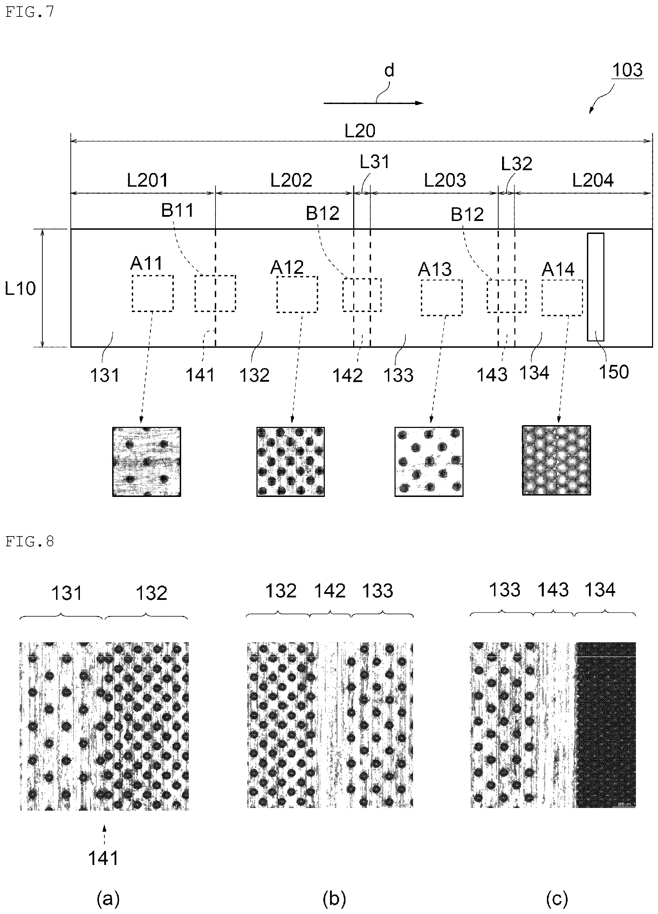

Referring to Fig. 7 and Fig. 8, a description will be given of the examination kit of the present embodiment. A description will be given of the points of difference with the first embodiment, the main point of difference being in the structure of the membrane carrier 103 and description of the same configuration and functions will not be repeated as appropriate.

Fig. 7 is a plan view schematically showing the membrane carrier 103. Fig. 8 shows an enlarged image of the boundaries between each adjacent region. Fig. 8(a) is an image of a first boundary 141 between the first fine structure region 131 and the second fine structure region 132.

As shown in the figures, the membrane carrier 103 has a rectangular shape with a predetermined length L10 and a width L20. The membrane carrier 103 is provided with, from the left side, a first fine structure region 131 (width L201), a second fine structure region 132 (width L202), a third fine structure region 133 (width L203), and a fourth fine structure region 134 (width L204) . As in the first embodiment, in these regions, the looseness and density of the protrusions in the fine structure are different, resulting in different speeds due to capillary action.

Specifically, the first fine structure region 131 is set to be the loosest (region A11), next, the third fine structure region 133 is the second loosest (region A13), the second fine structure region 132 is the third loosest (region A12), and the fourth fine structure region 134 is the densest (region A14). In addition, the fourth fine structure region 134 is provided with a solid-phase part 150.

In addition, a buffer region with a predetermined width L31 is provided at a second boundary 142 between the second fine structure region 132 and the third fine structure region 133. A fine structure (that is, the protrusions) is not provided in the buffer region. Similarly, a buffer region of a predetermined width L32 is provided at the third boundary 143 between the third fine structure region 133 and the fourth fine structure region 134. By providing such a buffer region, it is possible to absorb differences in the amount of solution transported in each region and prevent backflow or the like from being generated. For example, in the second fine structure region 132 and the third fine structure region 133, the fine structure of the third fine structure region 133 on the downstream side is loose. Accordingly, the solution has a higher movement speed in the second fine structure region 132. As a result, when there is no buffer region at the second boundary 142, backflow may occur depending on the amount of solution being deployed. However, as in the present embodiment, by providing a buffer region in which no capillary force is generated, it is possible to prevent backflow from being generated due to the movement speed of the solution and the amount of solution deployed.

### <Third Embodiment>

In the present embodiment, a description will be given of six examples of structures that prevent solution backflow. Here, a cross-sectional view of a partial region with the configuration corresponding to the membrane carriers 3 and 103 described above is extracted and explained, but the explanation also applies to other regions.

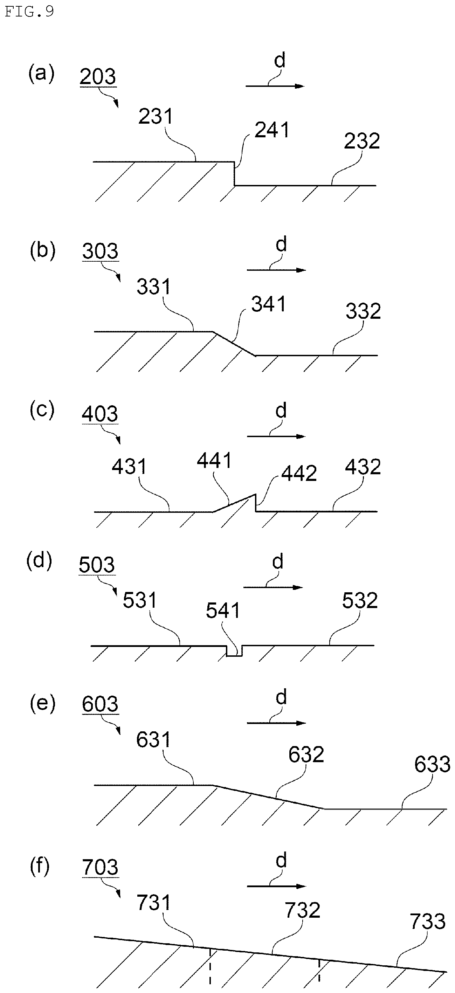

In a membrane carrier 203 shown in Fig. 9(a), a step portion 241 is provided at the boundary between a first fine structure region 231 and a second fine structure region 232 such that the second fine structure region 232 side is lower.

In a membrane carrier 303 shown in Fig. 9(b), an inclined portion 341 is provided at the boundary between a first fine structure region 331 and a second fine structure region 332, so as to be lower in the downstream direction. The inclined portion 341 may be a buffer region without protrusions or may be a fine uneven structure having protrusions.

In a membrane carrier 403 shown in Fig. 9(c), an inclined portion 441 is provided at the boundary between a first fine structure region 431 and a second fine structure region 432, so as to be lower in the upstream direction. The boundary between the inclined portion 441 and the second fine structure region 432 is a step portion 442.

In a membrane carrier 503 shown in Fig. 9(d), a recess 541 is provided at the boundary between a first fine structure region 531 and a second fine structure region 532.

In a membrane carrier 603 shown in Fig. 9(e), a first fine structure region 631 and a third fine structure region 633 are formed to be horizontal, but a second fine structure region 632 has an incline that is lower to the downstream side.

In a membrane carrier 703 shown in Fig. 9(f), a first fine structure region 731, a second fine structure region 732, and a third fine structure region 733 all have an inclination which is lower to the downstream side. In the configuration shown here, all have the same inclination angle; however, the inclination angle may be different in each region.

It is possible to combine the configurations in Fig. 9(a) to 9(f) described above as appropriate to make the desired membrane carrier and it is possible to realize the optimal flow channel and solution movement speed according to the type and amount of solution to be deployed.

Although the embodiments of the present invention were described with reference to the drawings, these are examples of the present invention and it is also possible to adopt various configurations (modified examples) other than those described above. For example, although the flow channel 2 was provided as a fine structure (fine uneven structure) of the membrane carrier 3 on a substrate formed of resin, it is possible to adopt various configurations, materials, and the like as long as it is possible to provide fine structures (fine uneven structures) with different looseness and density in regions of the flow channel 2.

### <Summary of Embodiments>

The characteristics of the present invention will be briefly summarized as follows.
(1) An examination device (examination kit) includes a flow channel provided on a substrate formed of resin and transporting a liquid sample from one end side to the other end side, a solid-phase part provided on the other end side of the flow channel, in which an antibody is set to a solid-phase, a detection unit provided with an electrode portion to detect a reaction of the liquid sample with respect to the antibody, a fine uneven structure having a plurality of protrusions formed integrally with the flow channel, wherein the fine uneven structure has a first uneven portion in which the plurality of protrusions are provided relatively loosely, and a second uneven portion in which the plurality of protrusions are provided relatively densely, and the first uneven portion and second uneven portion are provided further toward the one end side of the flow channel than the solid-phase part, and an introduction portion for introducing the liquid sample into the flow channel, wherein the liquid sample is formed of a plurality of types of solutions, and the introduction portion is provided at a plurality of locations according to the plurality of types of solutions; the detection unit is provided further toward the other end side of the flow channel than the solid-phase part; and wherein the first uneven portion and the second uneven portion are adjacent, a ratio (P1/P2) of a pitch (P1) between the protrusions in the first uneven portion and a pitch (P2) between the protrusions in the second uneven portion is 1.1 or more and 5 or less.
(2) The first uneven portion may be provided further toward the one end side of the flow channel than the second uneven portion.
(3) A buffer region in which no protrusion is provided may be included at the boundary between the first uneven portion and the second uneven portion.
(4) A step or an incline may be provided at the boundary between the first uneven portion and the second uneven portion and a region on the first uneven portion side of the step or the incline may be higher than the region on the second uneven portion side.
(5) A recess region provided with recesses may be included at the boundary between the first uneven portion and second uneven portion.
(6) A region in which the protrusions are provided in a diamond-shaped lattice may be included.
(7) A region in which the protrusions are provided in a regular lattice pattern may be included.
(8) The protrusions may be provided as cones.
(9) The electrode portion may be formed on the protrusions of the fine uneven structure, the maximum peak height Rp of the roughness curve of the electrode portion may be 0.005 µm or more and 10 µm or less, and the average length RSm of the roughness curve elements may be 0.01 µm or more and 15 µm or less.
(10) The electrode portion may have a conductor film layer formed by at least one of sputtering, vacuum evaporation, laser ablation, and CVD of a conductor substance on the protrusion of the fine uneven structure.
(11) The electrode portion may have a printed layer of paste including conductor particles on the protrusion of the fine uneven structure.
(12) The electrode portion may have a working electrode and a counter electrode separated from the working electrode and the working electrode may be provided at the same position as the counter electrode or on the upstream side from the counter electrode in the flow channel direction.
(13) The counter electrode may be provided over the entire width direction of the flow channel.
(14) The working electrode may be provided over the entire width direction of the flow channel.
(15) The working electrode may be formed as a comb-shaped electrode.
(16) The electrode portion may further have a reference electrode.
(17) A detection method detects the reaction of a liquid sample with respect to an antibody using the detection device described above.

### [Examples]

A specific description will be given of the present invention using Examples (Examples 1 and 2), but the present invention is not limited to these Examples. In the following Examples, experiments were conducted to evaluate the control of solution flow velocity when a fine structure was formed with a plurality of regions of different looseness and density in the membrane carrier.

### [Example 1]

A description will be given of this Example regarding an experiment for quantitative evaluation of the form of the solution swapping when a tricolor aqueous solution is deployed on the membrane carrier.

### 1. Experiment

(1) In order to confirm the technique of controlling solution deployment by changing the fine structure, in the membrane carrier 3 with the configuration shown in Fig. 2 of the first embodiment, the flow channel 2 in which three continuous regions in which the distances between the protrusions 8 of the first fine structure region 31, the second fine structure region 32, and the third fine structure region 33 were 25 µm, 15 µm, and 2 µm, respectively, was produced in polycarbonate (PC-2151 manufactured by Teijin Ltd.). The production conditions (thermal imprinting step) are as follows.

### <Thermal Imprinting Step (Fine Structure Transfer)>

The fine structure of the mold surface was transferred to the surface of a film-like base material formed of thermoplastic plastic by the following thermal imprinting step. In the thermal imprinting step, X-300 manufactured by SCIVAX was used. In the thermal imprinting step, the surface of the mold with the fine structure (a plurality of recesses) was applied to a film-like base material made of thermoplastic plastic and the mold and base material were pressed while being heated. The molding temperature was 180°C. The applied pressure was 5.5 MPa. The transfer time was 5 minutes. After the fine structure transfer, the mold and base material were cooled to 140°C while pressure was applied to the mold and base material. The pressure was released after cooling. The membrane carrier of Example 1 was obtained by the above thermal imprinting step. The membrane carrier had a surface that included a plurality of circular cones (fine structures) and flat portions. The shape and size of the protrusions (circular cones) on the surface of the membrane carrier matched the shape and size of the recesses (inverted circular cones) formed on the mold.

The protrusion 8 is a circular cone structure with a diameter 4 and a height 6 which are both 30 µm.

The length L1 of the membrane carrier 3 is 5 mm and respective widths L21 to L23 of the first to third fine structure regions 31 to 33 are each 20 mm.

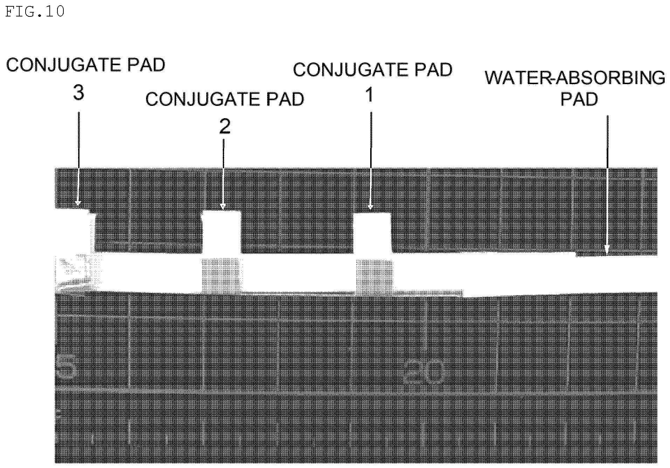

(2) A water-absorbing pad used in Navi-Flu was attached to the edge of the region of the third fine structure region 33, so as to overlap therewith by 5 mm. Furthermore, the conjugate pads used in Navi-Flu were fixed at the points where the distance between the fine structures changed (positions corresponding to the first boundary 41 and the second boundary 42 in Fig. 2) and test specimens were prepared. Fig. 10 shows a photograph of the prepared test specimen.

(3) The conjugate pads were numbered "1", "2", and "3" in order of proximity to a water-absorbing pad and aqueous solutions of the compositions shown in Table 1 were dropped on each pad. The amount of dropped solution was determined in consideration of the distance to the water-absorbing pad and the amount trapped in the conjugate pads during deployment. In order to simplify the operation and result verification of this experiment, drops were added to the pads in the order of "1", "2", and "3" every 10 seconds.

**[Table 1]**

| Table 1: Aqueous solution composition, droplet amount | | | |
|---|---|---|---|
| | Red food coloring and concentration [mg/mL] | Triton X-100 Concentration | Droplet amount [µL] |
| Drop location 1 | Food coloring green (made by Kyoritsu Foods) 10 | 2% | 20 |
| Drop location 2 | Food coloring red (made by Kyoritsu Foods) 10 | | 35 |
| Drop location 3 | Food coloring blue (made by Kyoritsu Foods) 10 | | 60 |

(4) Video was taken of the manner in which the solution of each color was deployed and the color change at the midpoint between drop point 1 and the water-absorbing pad was analyzed as an image.

### 2. Results

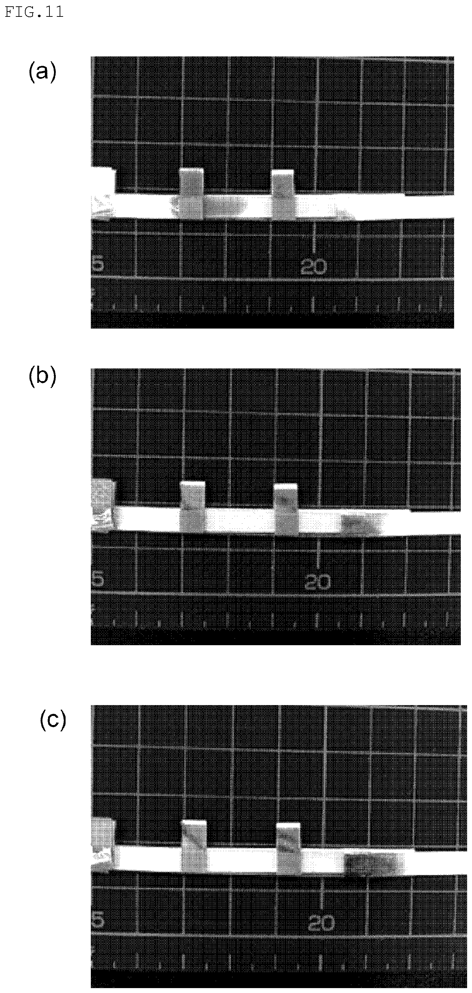

The imaged video was converted into images every 10 seconds and imported into image analysis software (software name "Image J"). Images at 30, 140, and 310 seconds after the start of the test are shown in Fig. 11. Fig. 11(a) is the image after 30 seconds, Fig. 11(b) is the image after 140 seconds, and Fig. 11(c) is the image after 310 seconds.

The measurement point was set at the midpoint between the drop point 1 and the water-absorbing pad and the RGB display data for that point was recorded. Next, the component ratio of each RGB color was calculated according to Equation 1. As an example, Table 2 shows the conversion data for each red (R), green (G), and blue (B) aqueous solution. (R or G or B component ratio) = (R or G or B value)/(R value + G value + B value)...

**[Table 2]**

| Table 2: Calculation from RGB values to component ratio | | | | | | | |
|---|---|---|---|---|---|---|---|
| | R value | G value | B value | | R component ratio | G component ratio | B component ratio |
| Red solution | 200 | 173 | 166 | → | 37% | 32% | 31% |
| Green solution | 137 | 194 | 161 | | 28% | 39% | 33% |
| Blue solution | 156 | 204 | 227 | | 27% | 35% | 39% |

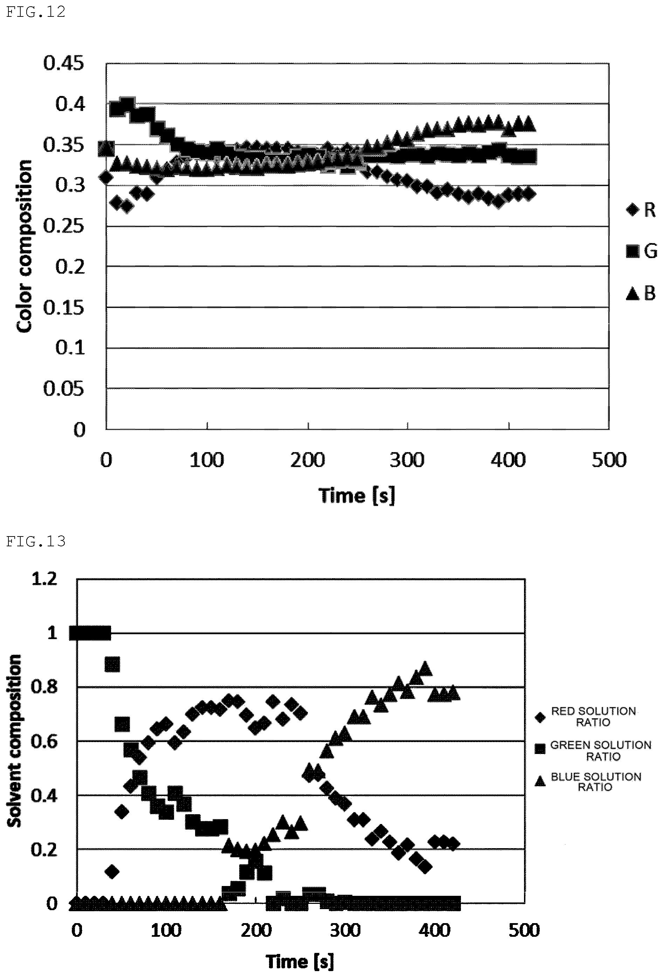

Fig. 12 shows a graph plotting the elapsed time against the RGB component ratio of the measurement point. From the results shown in Fig. 12, it is possible to confirm that it is possible to quantify the manner of change from green to red to blue as time passes.

Furthermore, in order to quantitatively evaluate the examination results and the flow manner, the following equation 2 was investigated as a method to quantitatively evaluate the mixing ratio. This was calculated by determining how the RGB component ratio of each measurement point was able to be realized according to the extent of mixing the component ratios of the monochromatic colors in Table 2. The solver function of the spreadsheet software Excel was used to minimize the error ε between the measured values and calculated values. ε= |R_r x+G_r y+B_r z-rl+|R_g x+G_g y+B_g z-g|+|R_b x+G_b y+B_b z-bl... x, y, and z are determined such that ε expressed in Equation 2 is minimized.

Here, R_r, R_g, and R_b are the R, G, and B component ratios of the red solution, respectively. G and B mean the same for the green solution and blue solution, respectively. r, g, and b are the measured values for the R, G, and B component ratios at the measurement point, respectively. x, y, and z are the mixing ratios of the red, green, and blue solutions at the measurement point, respectively, and were restricted such that x+y+z=1.

As a result of analyzing the data in Fig. 12 using Equation 2, it was possible to express the mixing ratios of each solution as shown in Fig. 13.

Based on these results, the temporal changes in the mixing ratios of the solutions were quantitatively evaluated by image analysis. That is, it was confirmed that, by appropriately setting the loose and dense state of the fine structure (protrusion spacing) in each region of the membrane carrier, it is possible to adjust the movement speed of the solution in the flow channels of the membrane carrier and to deploy a plurality of solutions.

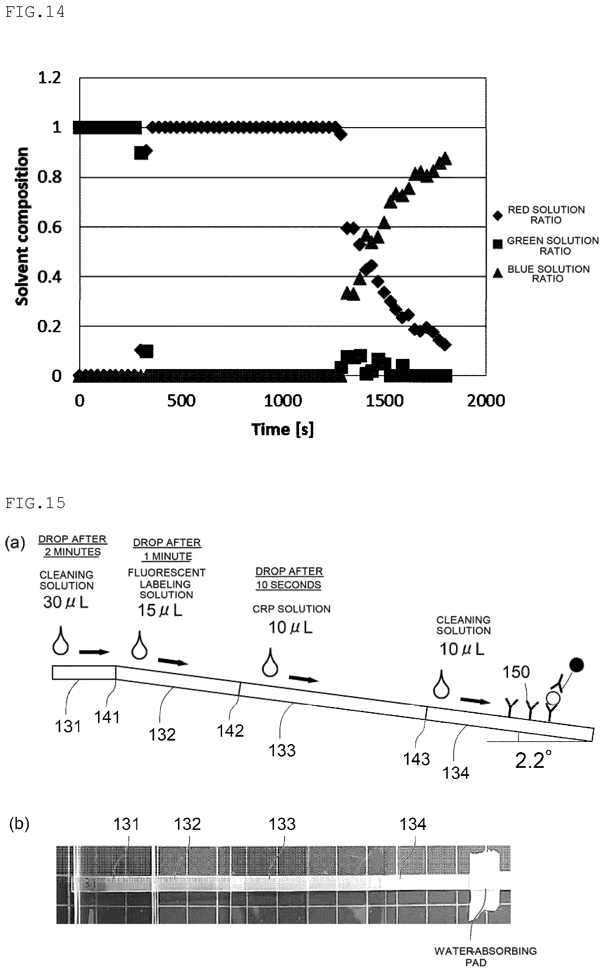

The results of a similar test and evaluation carried out using nitrocellulose instead of imprinted sheets (Comparative Example) are shown in Fig. 14. In the case of the imprinted sheet, it is possible to observe the solution in the flow channel from directly above because the structure (protrusion) is a circular cone, but the results are to be considered bearing in mind that nitrocellulose is a non-woven fabric and only the color change on the outermost surface is able to be observed.

Compared to Fig. 13 (imprinted sheet Example), it is clear that the test times are very different. This was a result of the difference in the deployment flow velocity of the imprinted sheet and nitrocellulose and it was possible to confirm the advantage of the imprinted sheet with a larger flow velocity in terms of quickly replacing the deployed solutions. Although it is possible to shorten the test time by adjusting the total length of the nitrocellulose, the risk of mixing solutions dropped at the same time is increased in such a case. In practice, in a test in which the total length and the amount of the dropped solution were halved, the solutions were mixed. Although it is potentially possible to produce a device with a structure designed to have a short determination time and no mixing of solutions, it is not possible to perform adjustment of the flow velocity or the like, and the design freedom is low. On the other hand, in a case where the membrane carrier is formed of imprinted sheets as shown in Example 1, there is a high degree of freedom in adjusting the flow velocity and the like.

### 3. Summary

According to Example 1, in the case of the imprinted sheet membrane carrier, it is possible to control the flow velocity by adjusting the fine structure and material and it is possible to respond flexibly to market needs.

### [Example 2]

### 1. Experiment

(1) To confirm the technique of controlling solution deployment by changing the fine structure, for the membrane carrier 103 of the second embodiment with the configuration shown in Fig. 7, a flow channel having four regions in which the distances between the vertices of the protrusions 8 of the first fine structure region 131, the second fine structure region 132, the third fine structure region 133, and the fourth fine structure region 134 were 105 µm, 60 µm, 80 µm, and 30 µm, respectively, was produced in polycarbonate (PC-2151 manufactured by Teij in Ltd.). The production conditions are the same as in Example 1. Fig. 15 (a) shows a schematic view in which the flow channel is seen from the side and Fig. 15(b) shows a top surface view (photo) of the test specimen produced in practice.

The protrusion 8 is a circular cone structure with the diameter 4 and the height 6 which are both 32 µm.

The length L1 of the membrane carrier 103 is 5 mm, the width L201 of the first fine structure region 131 = 15 mm, the width L202 of the second fine structure region 132 = 30 mm, the width L203 of the third fine structure region 133 = 45 mm, and the width L204 of the fourth fine structure region 134 = 40 mm.

In a side surface view, the second fine structure region 132, the third fine structure region 133, and the fourth fine structure region 134 are inclined at an inclination angle of 2.2°.

The first boundary 141 is a continuous boundary between the first fine structure region 131 and the second fine structure region 132 without a buffer region.

The second boundary 142 is the width L31 = 0.15 mm of the buffer region (unprocessed region).

The third boundary 143 is the width L32 = 0.20 mm of the buffer region (unprocessed region).

### (2) Experiment 1 (RGB Image Analysis):

In Experiment 1, using the same experiment and analysis method as in Example 1, the color change at a predetermined point from the RGB component was analyzed to quantitatively confirm the manner in which the liquid was replaced.

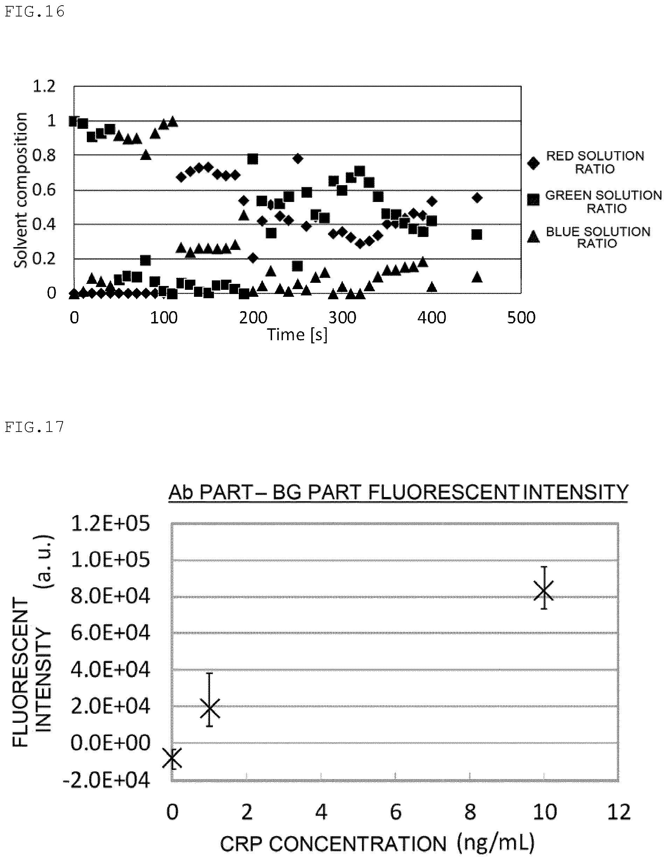

Specifically, the color change at a point 5 mm from the most downstream part (the right-hand edge of the fourth fine structure region 134 shown in the figure) was analyzed from the RGB components. That is, the solution mixing ratio during the deployment was evaluated based on the RGB component ratio of each solution. Fig. 16 shows a graph of the evaluation results. This corresponds to Fig. 13 of Example 1 and it was confirmed that, as time passed, the components with larger ratios changed from the green component (G), the red component (R), and the blue component (B).

### (3) Experiment 2 (CRP Detection Performance Evaluation):

In this experiment, as shown in Fig. 15(a), as a solution deployment step, next, 10 µL of a cleaning solution was dropped into the fourth fine structure region 134, 10 seconds later, 10 µL of a CRP solution was dropped into the third fine structure region 133, 1 minute later, 15 µL of a fluorescent labeling solution was dropped into the second fine structure region 132, and, finally, 2 minutes later, 30 µL of a cleaning solution was dropped into the first fine structure region 131. The fluorescence intensity was measured 10 minutes after the last solution (cleaning solution) was dropped.

The solutions used were as follows.
Cleaning solution: PBS containing 2 wt% Triton X-100
CRP solution: Solution of the cleaning solution and a CRP solution mixed at a predetermined concentration
Fluorescent labeling solution: Solution of a mixture of the cleaning solution and a fluorescent-labeled anti-CRP antibody solution at an antibody concentration of 30 µg/mL.

Fig. 17 shows the fluorescence intensity for each CRP concentration. In addition, Fig. 18 shows images of the observed fluorescence intensity. Fig. 18(a) is when the CRP concentration was 0 ng/mL and Fig. 18 (b) is when the CRP concentration was 10 ng/mL. From the results shown in Fig. 17 and Fig. 18, at CRP concentrations above 1 ng/mL, the CRP concentration-dependent fluorescence intensity was obtained. Thus, by applying the membrane carrier having a plurality of fine structures as described above to electrochemical detection (electrochemical immunochromatography), it is possible to electrically measure the desired detection target substance.

### [Example 3]

### 1. Experiment

### (1) Test Specimen (Membrane Carrier 103)

In Example 3, for the purpose of making the test system closer to actual use conditions, the test conditions of Experiment 2 in Example 2 were partially changed and a CRP detection test was carried out in which human serum was mixed with the sample solution.

In the structure of the test specimen (membrane carrier 103) corresponding to Fig. 7, a flow channel having four regions in which the distances between the vertices of the protrusions 8 of the first fine structure region 131, the second fine structure region 132, the third fine structure region 133, and the fourth fine structure region 134 were 100 µm, 60 µm, 95 µm, and 30 µm, respectively, was produced in polycarbonate (PC-2151 manufactured by Teijin Ltd.). The production conditions are the same as in Example 1 and Example 2.

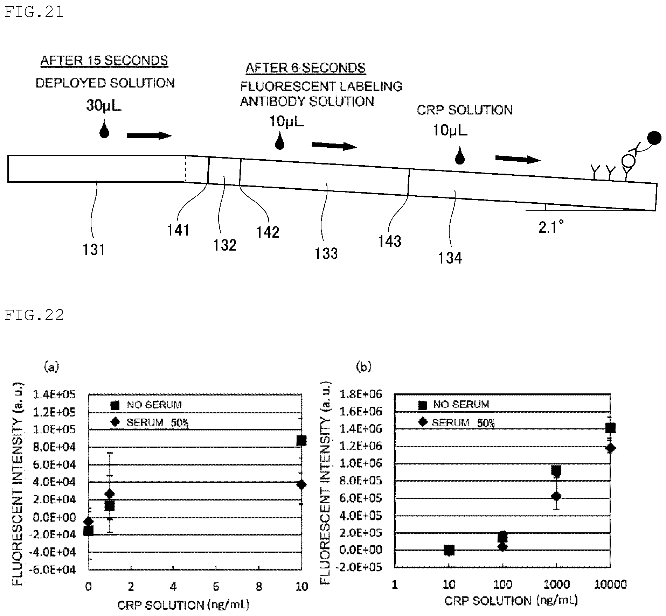

Fig. 21 shows a schematic view of the test specimen (flow channel) seen from the side surface. Thirty-six samples were prepared as test specimens. The protrusion 8 is a circular cone structure with the diameter 4 and the height 6 which are both 32 µm.

The length L1 of the membrane carrier 103 is 5 mm, the width L201 of the first fine structure region 131 = 36.95 mm, the width L202 of the second fine structure region 132 = 5 mm, the width L203 of the third fine structure region 133 = 40 mm, and the width L204 of the fourth fine structure region 134 = 40 mm.

In a side surface view, the second fine structure region 132, the third fine structure region 133, and the fourth fine structure region 134 are inclined at an inclination angle of 2.1°.

The first boundary 141 is a continuous boundary between the first fine structure region 131 and the second fine structure region 132 without a buffer region.

At the second boundary 142, the width L31 of the buffer region (unprocessed region) = 0.15 mm.

At the third boundary 143, the width L32 of the buffer region (unprocessed region) = 0.15 mm.

### (2) Antibody Solid Phase

1 µL of an anti-CRP antibody solid-phase solution was dropped at a position of 17.5 mm from the most downstream end of the test specimen and dried at 45°C in the atmosphere for 1 hour to set 25 ng of the anti-CRP antibody as a solid-phase.

As shown in Fig. 21, an inclination of 2.1° was provided in the flow channel and a CRP solution, a fluorescent-labeled anti-CRP antibody solution (in the deployed solution, 45 µg/mL fluorescent-labeled anti-CRP antibody concentration), and a deployed solution (PBS containing 2 wt% Triton X-100) were deployed in order from different drop points. The composition of the CRP solution used is shown in Table 3. The deployed solution amount and the interval between solution drops are shown in Table 4. In addition, each test was carried out with n = 3.

Ten minutes after all solutions were deployed, the water-absorbing pad and the imprinted sheet (the membrane carrier 103) were separated to prevent backflow of the solution and then the fluorescence intensity of the antibody solid phase was measured.

### 2. Results

The fluorescence intensity measurement results for each test are shown in Fig. 22. The fluorescence intensity shown here is the value obtained by subtracting the background fluorescence intensity in the periphery from the fluorescence intensity of the antibody solid-phase part. In Fig. 22(a), the exposure time for the fluorescence intensity measurement was set to 1 second and, in Fig. 22(b), the exposure time was set to 1/6 second.

It was possible to achieve an equivalent minimum detection sensitivity and a measurement range of three digits or more (detection range) regardless of the presence or absence of serum.

The fluorescence intensity tended to decrease with the presence of serum. It is assumed that the protein in the serum (up to 80 mg/mL) inhibited the reaction between the antibody and CRP.

**[Table 3]**

| Table 3: CRP solution composition | | | | | |
|---|---|---|---|---|---|
| Serum mixed | Final CRP concentration | Buffer solution | Ratio of buffer solution in solution | CRP concentration in buffer solution | Human serum ratio in solution |
| No | 0 | Deployed solution (2w/v% PBS containing Triton X-100) | 100% | 0 | - |
| | 1 ng/mL | | | 1 ng/mL | |
| | 10 ng/mL | | | 10 ng/mL | |
| | 100 ng/mL | | | 100 ng/mL | |
| | 1 µg/mL | | | 1 µg/mL | |
| | 10 µg/mL | | | 10 µg/mL | |
| Yes | 0 | 4w/v% PBS containing Triton X-100 | 50% | 0 | 50% |
| | 1 ng/mL | | | 2 ng/mL | |
| | 10 ng/mL | | | 20 ng/mL | |
| | 100 ng/mL | | | 200 ng/mL | |
| | 1 µg/mL | | | 2 µg/mL | |
| | 10 µg/mL | | | 20 µg/mL | |

**[Table 4]**

| Table 4: Examination protocol | | | | |
|---|---|---|---|---|
| 1. CRP solution | Interval | 2. Fluorescent labeled anti-CRP: antibody solution | Interval | : 3. Deployed solution |
| Fourth fine structure region 10 µL | 6 seconds | Third fine structure region 10 µL | 10 seconds | First fine structure region 30 uL |

### [Example 4]

In Example 4, electrochemical detection tests were carried out on the imprinted sheet based on the results of Examples 1 to 3. In this Example, from the viewpoint of confirming whether it was possible to perform the electrochemical detection appropriately or not, all solutions were dropped at the same position in the fourth fine structure region 134 with different drop timings.

### 1. Experiment

### (1) Test Specimen (Membrane Carrier 103)

A membrane carrier 103 with the same structure as the test specimen produced in Example 3 was prepared. The length L1 of the membrane carrier 103 is 5 mm, the width L201 of the first fine structure region 131 = 36.95 mm, the width L202 of the second fine structure region 132 = 5 mm, the width L203 of the third fine structure region 133 = 40 mm, and the width L204 of the fourth fine structure region 134 = 40 mm.

### (2) Electrode Portion (Working electrode and Counter Electrode)

An imprinted sheet was attached to a substrate formed by attaching polyimide tape on a SUS plate and gold was vacuum evaporated through a mask processed into an electrode shape as the electrode portion 20.

The electrode shape is as follows.
Working electrode...1 mm x 5 mm (flow channel width)
Counter electrode...3 mm x 5 mm (flow channel width)
Gap between electrodes...0.5 mm
Electrode position...the downstream end of the counter electrode is at a position 15.5 mm from the most downstream end of the flow channel.

### (3) Antibody Solid Phase

In the same manner as in Example 3, 1 µL of an anti-CRP antibody solid-phase solution was dropped on a position 5 mm upstream position from the working electrode and dried at 45°C in the atmosphere for 1 hour to set 25 ng of the anti-CRP antibody as a solid-phase.

### (4) Measuring Device

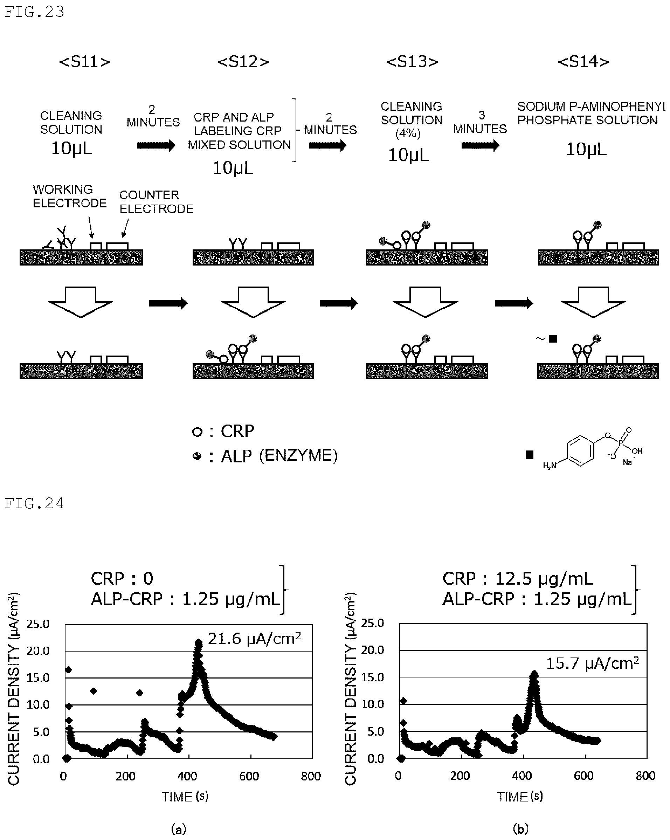

Between the electrode portion 20 (working electrode and counter electrode) and the substrate was made to be conductive with silver paste (Dotite D-550) and the substrate was clipped between the alligator clips of an electrochemical measuring device (1252A manufactured by Solartron). The solution was deployed by applying a potential of +50 mV between the working electrode and the counter electrode and the current values were plotted against time (refer to Fig. 24).

### (5) Solution Drop Timing and Dropped Solution

Fig. 23 is a chart showing the solution drop timing in Example 4. First, 10 µL of cleaning solution was dropped (first step S11). Two minutes after the first step S11, 10 µL of a CRP and ALP labeled CRP mixed solution was dropped (second step S12). Furthermore, two minutes after the second step S12, 10 µL of a cleaning solution (4%) was dropped (third step S13). Finally, three minutes after the third step S13, 10 µL of sodium p-aminophenyl phosphate solution was dropped (fourth step S14).

The dropped solutions were as follows.
Cleaning solution... PBS containing 2 wt% Triton X-100
ALP labeled CRP...commercially available CRP labeled with a labeling kit (LK13 by Dojindo Laboratories)
CRP and ALP labeling CRP mixed solution... Suspend CRP and ALP labeled CRP in cleaning solution to a specified concentration
Cleaning solution (4%) ... PBS containing 4 wt% Triton X-100
Sodium p-aminophenylphosphate solution...Dissolved sodium p-aminophenyl phosphate to a concentration of 5 mM in cleaning solution

### 2. Results

Fig. 24 shows the measurement results. Fig. 24(a) shows the results when the CRP concentration of the ALP labeled CRP mixed solution was 0 µg/mL and the ALP-CRP concentration was 1.25 µg/mL, and Fig. 24(b) shows the results when the CRP concentration of the ALP labeled CRP mixed solution was 12.5 µg/mL and the ALP-CRP concentration was 1.25 µg/mL. As shown in the figure, current values reflecting the drop timing and CRP concentration of the solution were detected.

This application claims priority based on Japanese Application No. 2020-079367, filed April 28, 2020.

### REFERENCE SIGNS LIST

2 Flow channel
3, 103, 203, 303, 403, 503, 603, 703 Membrane carrier
3x Cleaning solution zone
3y Detection zone
3z Droplet zone
8 Protrusion
18 Examination kit
18a Housing
18b First opening
18c Second opening
18d Third opening
20 Electrode portion
21 Measuring device
25 Working electrode
26 Counter electrode
27 Reference electrode
31, 131, 231, 331, 431, 531, 631, 731 First fine structure region
32, 132, 232, 332, 432, 532, 632, 732 Second fine structure region
33, 133 Third fine structure region
134 Fourth fine structure region
41, 141, 241 First boundary
42, 142 Second boundary
143 Third boundary
50, 150 Solid-phase part
51 Antibody
341, 441 Inclined portion
541 Recess

## Claims

1. A detection device comprising:
(i) a flow channel provided on a substrate formed of resin and transporting a liquid sample from one end side to the other end side;
(ii) a solid-phase part provided on the other end side of the flow channel, in which an antibody is set to a solid-phase;
(iii) a detection unit provided with an electrode portion to detect a reaction of the liquid sample with respect to the antibody;
(iv) a fine uneven structure having a plurality of protrusions formed integrally with the flow channel,
wherein the fine uneven structure has a first uneven portion in which the plurality of protrusions are provided relatively loosely, a second uneven portion in which the plurality of protrusions are provided relatively densely, and the first uneven portion and second uneven portion are provided further toward the one end side of the flow channel than the solid-phase part; and
(v) an introduction portion for introducing the liquid sample into the flow channel,
wherein the liquid sample is formed of a plurality of types of solutions, and the introduction portion is provided at a plurality of locations according to the plurality of types of solutions;
the detection unit is provided further toward the other end side of the flow channel than the solid-phase part; and
wherein the first uneven portion and the second uneven portion are adjacent, a ratio (P1/P2) of a pitch (P1) between the protrusions in the first uneven portion and a pitch (P2) between the protrusions in the second uneven portion is 1.1 or more and 5 or less.

2. The detection device according to claim 1,
wherein the first uneven portion is provided further toward the one end side of the flow channel than the second uneven portion.

3. The detection device according to claim 1 or 2,
wherein the detection device has a buffer region in which no protrusion is provided at a boundary of the first uneven portion and the second uneven portion.

4. The detection device according to any one of claims 1 to 3,
wherein a step or an incline is provided at a boundary between the first uneven portion and the second uneven portion, and
a region on a first uneven portion side of the step or the incline is higher than a region on a second uneven portion side.

5. The detection device according to any one of claims 1 to 4,
wherein the detection device has a recess region in which a recess is provided at a boundary between the first uneven portion and the second uneven portion.

6. The detection device according to any one of claims 1 to 5,
wherein the detection device has a region in which the protrusions are provided in a diamond-shaped lattice.

7. The detection device according to any one of claims 1 to 6,
wherein the detection device has a region in which the protrusions are provided in a regular lattice pattern.

8. The detection device according to any one of claims 1 to 7,
wherein the protrusion is provided as a cone.

9. The detection device according to any one of claims 1 to 8,
wherein the electrode portion is formed on the protrusion of the fine uneven structure, a maximum peak height Rp of a roughness curve of the electrode portion is 0.005 µm or more and 10 µm or less, and an average length RSm of a roughness curve element is 0.01 µm or more and 15 µm or less.

10. The detection device according to any one of claims 1 to 9,
wherein the electrode portion has a conductor film layer formed of a conductor substance on the protrusion of the fine uneven structure by at least one type of sputtering, vacuum evaporation, laser ablation, or CVD.

11. The detection device according to any one of claims 1 to 9,
wherein the electrode portion has a printed layer of paste including conductor particles on the protrusion of the fine uneven structure.

12. The detection device according to any one of claims 1 to 11,
wherein the electrode portion has a working electrode and a counter electrode separated from the working electrode, and
the working electrode is provided at the same position as the counter electrode or on an upstream side from the counter electrode in a flow channel direction.

13. The detection device according to claim 12,
wherein the counter electrode is provided over an entire width direction of the flow channel.

14. The detection device according to claim 12 or 13,
wherein the working electrode is provided over the entire width direction of the flow channel.

15. The detection device according to any one of claims 12 to 14,
wherein the working electrode is configured as a comb-shaped electrode.

16. The detection device according to any one of claims 12 to 15,
wherein the electrode portion further has a reference electrode.

17. A detection method for detecting a reaction of a liquid sample with respect to an antibody using the detection device according to any one of claims 1 to 16.

## Patentansprüche

1. Nachweisvorrichtung, die umfasst:
(i) einen Fließkanal, der auf einem aus Harz geformten Substrat bereitgestellt ist und eine flüssige Probe von einer Endseite zu der anderen Endseite transportiert;
(ii) einen Festphasenteil, der an der anderen Endseite des Fließkanals bereitgestellt ist und in dem ein Antikörper auf eine Festphase aufgebracht ist;
(iii) eine Nachweiseinheit, die mit einem Elektrodenabschnitt bereitgestellt ist, zum Nachweisen einer Reaktion der flüssigen Probe hinsichtlich des Antikörpers;
(iv) eine feine, unebene Struktur mit einer Vielzahl von Vorsprüngen, die integral mit dem Fließkanal ausgebildet sind,
wobei die feine, unebene Struktur einen ersten unebenen Abschnitt, in dem die Vielzahl von Vorsprüngen relativ lose bereitgestellt ist, einen zweiten unebenen Abschnitt, in dem die Vielzahl von Vorsprüngen relativ dicht bereitgestellt ist, aufweist und der erste unebene Abschnitt und der zweite unebene Abschnitt weiter in Richtung der einen Endseite des Fließkanals bereitgestellt sind als der Festphasenteil; und
(v) einen Einbringabschnitt zum Einbringen der flüssigen Probe in den Fließkanal,
wobei die flüssige Probe aus einer Vielzahl von Arten von Lösungen gebildet ist und der Einbringabschnitt an einer Vielzahl von Stellen entsprechend der Vielzahl von Arten von Lösungen bereitgestellt ist;
die Nachweiseinheit weiter in Richtung der anderen Endseite des Fließkanals bereitgestellt ist als der Festphasenteil; und
wobei der erste unebene Abschnitt und der zweite unebene Abschnitt benachbart sind, ein Verhältnis (P1/P2) eines Abstandes (P1) zwischen den Vorsprüngen in dem ersten unebenen Abschnitt und eines Abstandes (P2) zwischen den Vorsprüngen in dem zweiten unebenen Abschnitt 1,1 oder mehr und 5 oder weniger beträgt.

2. Nachweisvorrichtung gemäß Anspruch 1,
wobei der erste unebene Abschnitt weiter in Richtung der einen Endseite des Fließkanals bereitgestellt ist als der zweite unebene Abschnitt.

3. Nachweisvorrichtung gemäß Anspruch 1 oder 2,
wobei die Nachweisvorrichtung einen Pufferbereich, in dem kein Vorsprung bereitgestellt ist, an einer Grenze des ersten unebenen Abschnitts und des zweiten unebenen Abschnitts aufweist.

4. Nachweisvorrichtung gemäß einem jeglichen der Ansprüche 1 bis 3,
wobei eine Stufe oder eine Neigung an einer Grenze zwischen dem ersten unebenen Abschnitt und dem zweiten unebenen Abschnitt bereitgestellt ist, und
ein Bereich auf einer ersten unebenen Abschnittsseite der Stufe oder der Neigung höher ist als ein Bereich auf einer zweiten unebenen Abschnittsseite.

5. Nachweisvorrichtung gemäß einem jeglichen der Ansprüche 1 bis 4,
wobei die Nachweisvorrichtung einen Aussparungsbereich, in dem eine Aussparung bereitgestellt ist, an einer Grenze zwischen dem ersten unebenen Abschnitt und dem zweiten unebenen Abschnitt aufweist.

6. Nachweisvorrichtung gemäß einem jeglichen der Ansprüche 1 bis 5,
wobei die Nachweisvorrichtung einen Bereich aufweist, in dem die Vorsprünge in einem Gitter in Rautenform bereitgestellt sind.

7. Nachweisvorrichtung gemäß einem jeglichen der Ansprüche 1 bis 6,
wobei die Nachweisvorrichtung einen Bereich aufweist, in dem die Vorsprünge in einem regelmäßigen Gittermuster bereitgestellt sind.

8. Nachweisvorrichtung gemäß einem jeglichen der Ansprüche 1 bis 7,
wobei der Vorsprung als ein Konus bereitgestellt ist.

9. Nachweisvorrichtung gemäß einem jeglichen der Ansprüche 1 bis 8,
wobei der Elektrodenabschnitt auf dem Vorsprung der feinen, unebenen Struktur ausgebildet ist, eine maximale Peakhöhe Rp einer Rauheitskurve des Elektrodenabschnitts 0,005 µm oder mehr und 10 µm oder weniger beträgt und eine durchschnittliche Länge RSm eines Rauheitskurvenelements 0,01 µm oder mehr und 15 µm oder weniger beträgt.

10. Nachweisvorrichtung gemäß einem jeglichen der Ansprüche 1 bis 9,
wobei der Elektrodenabschnitt eine leitende Filmschicht aufweist, die aus einer leitenden Substanz auf dem Vorsprung der feinen, unebenen Struktur durch mindestens eine Art von Sputtern, Vakuumverdampfung, Laserablation oder CVD gebildet ist.

11. Nachweisvorrichtung gemäß einem jeglichen der Ansprüche 1 bis 9,
wobei der Elektrodenabschnitt eine gedruckte Schicht einer Paste, die leitende Partikel beinhaltet, auf dem Vorsprung der feinen, unebenen Struktur aufweist.

12. Nachweisvorrichtung gemäß einem jeglichen der Ansprüche 1 bis 11,
wobei der Elektrodenabschnitt eine Arbeitselektrode und eine von der Arbeitselektrode separierte Gegenelektrode aufweist, und
die Arbeitselektrode an der gleichen Position wie die Gegenelektrode oder an einer stromaufwärtigen Seite von der Gegenelektrode in einer Fließkanalrichtung bereitgestellt ist.

13. Nachweisvorrichtung gemäß Anspruch 12,
wobei die Gegenelektrode über eine gesamte Breitenrichtung des Fließkanals bereitgestellt ist.

14. Nachweisvorrichtung gemäß Anspruch 12 oder 13,
wobei die Arbeitselektrode über die gesamte Breitenrichtung des Fließkanals bereitgestellt ist.

15. Nachweisvorrichtung gemäß einem jeglichen der Ansprüche 12 bis 14,
wobei die Arbeitselektrode als eine Elektrode in Kammform konfiguriert ist.

16. Nachweisvorrichtung gemäß einem jeglichen der Ansprüche 12 bis 15,
wobei der Elektrodenabschnitt ferner eine Referenzelektrode aufweist.

17. Nachweisverfahren zum Nachweisen einer Reaktion einer flüssigen Probe hinsichtlich eines Antikörpers unter Verwendung der Nachweisvorrichtung gemäß einem jeglichen der Ansprüche 1 bis 16.

## Revendications

1. Dispositif de détection, comprenant :
(i) un canal d'écoulement ménagé sur un substrat formé de résine et transportant un échantillon liquide d'un côté d'extrémité à l'autre côté d'extrémité ;
(ii) une partie en phase solide ménagée à l'autre côté d'extrémité du canal d'écoulement, où un anticorps est mis en phase solide ;
(iii) une unité de détection dotée d'une partie d'électrode pour détecter une réaction de l'échantillon liquide par rapport à l'anticorps ;
(iv) une structure fine et irrégulière comportant une pluralité de saillies formées d'un seul tenant avec le canal d'écoulement,
dans lequel la structure fine et irrégulière comporte une première partie irrégulière où la pluralité de saillies est ménagée de manière relativement lâche, une seconde partie irrégulière où la pluralité de saillies est ménagée de manière relativement dense, et la première partie irrégulière et la seconde partie irrégulière sont ménagées plus loin vers l'un côté d'extrémité du canal d'écoulement que la partie en phase solide ; et
(v) une partie d'introduction permettant d'introduire l'échantillon liquide dans le canal d'écoulement,
dans lequel l'échantillon liquide est formé d'une pluralité de types de solutions, et la partie d'introduction est ménagée à une pluralité d'emplacements selon la pluralité de types de solutions ;
l'unité de détection est ménagée plus loin vers l'autre côté d'extrémité du canal d'écoulement que la partie en phase solide ; et
dans lequel la première partie irrégulière et la seconde partie irrégulière sont adjacentes, un rapport (P1/P2) d'un pas (P1) entre les saillies dans la première partie irrégulière et d'un pas (P2) entre les saillies dans la seconde partie irrégulière est de 1,1 ou plus et de 5 ou moins.

2. Dispositif de détection selon la revendication 1, dans lequel la première partie irrégulière est ménagée plus loin vers l'un côté d'extrémité du canal d'écoulement que la seconde partie irrégulière.

3. Dispositif de détection selon la revendication 1 ou 2, dans lequel le dispositif de détection comporte une région tampon où aucune saillie n'est ménagée au niveau d'une limite de la première partie irrégulière et de la seconde partie irrégulière.

4. Dispositif de détection selon l'une quelconque des revendications 1 à 3, dans lequel une marche ou une inclinaison est ménagée au niveau d'une limite entre la première partie irrégulière et la seconde partie irrégulière, et
une région sur un premier côté de partie irrégulière de la marche ou de l'inclinaison est plus haute qu'une région sur un second côté de partie irrégulière.

5. Dispositif de détection selon l'une quelconque des revendications 1 à 4, dans lequel le dispositif de détection comporte une région d'évidement où un évidement est ménagé au niveau d'une limite entre la première partie irrégulière et la seconde partie irrégulière.

6. Dispositif de détection selon l'une quelconque des revendications 1 à 5, dans lequel le dispositif de détection comporte une région où les saillies sont ménagées dans un réseau en forme de losange.

7. Dispositif de détection selon l'une quelconque des revendications 1 à 6,
dans lequel le dispositif de détection comporte une région où les saillies sont ménagées dans un motif en treillis régulier.

8. Dispositif de détection selon l'une quelconque des revendications 1 à 7, dans lequel la saillie est ménagée sous forme de cône.

9. Dispositif de détection selon l'une quelconque des revendications 1 à 8, dans lequel la partie d'électrode est formée sur la saillie de la structure fine et irrégulière, une hauteur de pic maximale Rp d'une courbe de rugosité de la partie d'électrode est de 0,005 µm ou plus et de 10 µm ou moins, et une longueur moyenne RSm d'un élément de courbe de rugosité est de 0,01 µm ou plus et de 15 µm ou moins.

10. Dispositif de détection selon l'une quelconque des revendications 1 à 9, dans lequel la partie d'électrode comporte une couche de film conducteur formée d'une substance conductrice sur la saillie de la structure fine irrégulière par au moins un type de pulvérisation cathodique, d'évaporation sous vide, d'ablation laser ou de CVD.

11. Dispositif de détection selon l'une quelconque des revendications 1 à 9, dans lequel la partie d'électrode comporte une couche imprimée de pâte comprenant des particules conductrices sur la saillie de la fine structure irrégulière.

12. Dispositif de détection selon l'une quelconque des revendications 1 à 11, dans lequel la partie d'électrode comporte une électrode de travail et une contre-électrode séparée de l'électrode de travail, et
l'électrode de travail est ménagée au niveau de la même position que la contre-électrode ou sur un côté en amont de la contre-électrode dans une direction de canal d'écoulement.

13. Dispositif de détection selon la revendication 12, dans lequel la contre-électrode est ménagée sur toute une direction de largeur du canal d'écoulement.

14. Dispositif de détection selon la revendication 12 ou 13, dans lequel l'électrode de travail est ménagée sur toute la direction de largeur du canal d'écoulement.

15. Dispositif de détection selon l'une quelconque des revendications 12 à 14, dans lequel l'électrode de travail est conçue comme une électrode en forme de peigne.

16. Dispositif de détection selon l'une quelconque des revendications 12 à 15, dans lequel la partie d'électrode comporte en outre une électrode de référence.

17. Procédé de détection destiné à détecter une réaction d'un échantillon liquide par rapport à un anticorps à l'aide du dispositif de détection selon l'une quelconque des revendications 1 à 16.
